# FASCICULE DE BREVET EUROPEEN

(11) **EP 2 576 828 B1**
(45) Date de publication et mention de la délivrance du brevet: **25.11.2015**
(21) Numéro de dépôt: 11728645.0
(22) Date de dépôt: 30.05.2011
(51) Int. Cl.: C12Q 1/68

(54) **PROCEDE DE GENOTYPAGE DE STAPHYLOCOCCUS AUREUS**
VERFAHREN ZUR GENOTYPISIERUNG VON STAPHYLOCOCCUS AUREUS
PROCESS OF GENOTYPING OF STAPHYLOCOCCUS AUREUS

(30) Priorité: 01.06.2010 FR 1054238
(43) Date de publication de la demande: 10.04.2013
(73) Titulaire: Centre Européen d'Expertise et de Recherche sur les Agents Microbiens CEERAM, 44240 La Chapelle sur Erdre (FR)
(72) Inventeur: LEBEAU, Benoît, F-44700 Orvault (FR); LOISY-HAMON, Fabienne, F-44700 Orvault (FR); SOBRAL, Daniel, F-78310 Maurepas (FR); POURCEL, Christine, F-91400 Orsay (FR)
(74) Mandataire: Le Cloirec, Claudine
(86) Numéro de dépôt international: PCT/FR2011/051233
(87) Numéro de publication internationale: WO 2011/151586

(56) Documents cités:
- US-A1- 2006 020 391
- US-A1- 2007 117 136
- FRANCOIS PATRICE ET AL: "Use of an automated multiple-locus, variable-number tandem repeat-based method for rapid and high-throughput genotyping of Staphylococcus aureus isolates", JOURNAL OF CLINICAL MICROBIOLOGY, AMERICAN SOCIETY FOR MICROBIOLOGY, WASHINGTON, DC, US, vol. 43, no. 7, 1 juillet 2005 (2005-07-01), pages 3346-3355, XP002419294, ISSN: 0095-1137, DOI: DOI:10.1128/JCM.43.7.3346-3355.2005
- POURCEL CHRISTINE ET AL: "Improved multiple-locus variable-number tandem-repeat assay for Staphylococcus aureus genotyping, providing a highly informative technique together with strong phylogenetic value.", JOURNAL OF CLINICAL MICROBIOLOGY OCT 2009 LNKD- PUBMED:19710277, vol. 47, no. 10, octobre 2009 (2009-10), pages 3121-3128, XP002612326, ISSN: 1098-660X cité dans la demande
- LEO M. SCHOULS ET AL: "Multiple-Locus Variable Number Tandem Repeat Analysis of Staphylococcus Aureus: Comparison with Pulsed-Field Gel Electrophoresis and spa-Typing", PLOS ONE, vol. 4, no. 4, 1 janvier 2009 (2009-01-01) , page E5082, XP55004719, ISSN: 1932-6203, DOI: 10.1371/journal.pone.0005082 cité dans la demande
- VERGNAUD GILLES ET AL: "Multiple locus variable number of tandem repeats analysis", METHODS IN MOLECULAR BIOLOGY, HUMANA PRESS INC, NJ, US, vol. 551, 1 janvier 2009 (2009-01-01), pages 141-158, XP009141801, ISSN: 1064-3745, DOI: DOI:10.1007/978-1-60327-999-4_12
- HOLMES A ET AL: "Comparison of two multilocus variable-number tandem-repeat methods and pulsed-field gel electrophoresis for differentiating highly clonal methicillin-resistant Staphylococcus aureus isolates", JOURNAL OF CLINICAL MICROBIOLOGY, AMERICAN SOCIETY FOR MICROBIOLOGY, WASHINGTON, DC, US, vol. 48, no. 10, 11 août 2010 (2010-08-11) , pages 3600-3607, XP009141862, ISSN: 0095-1137, DOI: DOI:10.1128/JCM.01039-10
- MARKOULATOS P ET AL: "MULTIPLEX POLYMERASE CHAIN REACTION: A PRACTICAL APPROACH", JOURNAL OF CLINICAL LABORATORY ANALYSIS, NEW YORK, NY, US, vol. 16, no. 1, 1 janvier 2002 (2002-01-01), pages 47-51, XP009003351, ISSN: 0887-8013, DOI: DOI:10.1002/JCLA.2058
- LISTA FLORIGIO ET AL: "Genotyping of Bacillus anthracis strains based on automated capillary 25-loci Multiple Locus Variable-Number Tandem Repeats Analysis", BMC MICROBIOLOGY, BIOMED CENTRAL, LONDON, GB, vol. 6, no. 1, 6 avril 2006 (2006-04-06), page 33, XP021014862, ISSN: 1471-2180, DOI: DOI:10.1186/1471-2180-6-33
- DENOEUD FRANCE ET AL: "Identification of polymorphic tandem repeats by direct comparison of genome sequence from different bacterial strains : a web-based resource", BMC BIOINFORMATICS, BIOMED CENTRAL, LONDON, GB, vol. 5, no. 1, 12 janvier 2004 (2004-01-12), page 4, XP021000621, ISSN: 1471-2105, DOI: DOI:10.1186/1471-2105-5-4
- SOBRAL DANIEL ET AL: "High Throughput Multiple Locus Variable Number of Tandem Repeat Analysis (MLVA) of Staphylococcus aureus from Human, Animal and Food Sources", PLOS ONE, vol. 7, no. 5, May 2012 (2012-05),

### SEQUENCE LISTING

<110> CENTRE EUROPEEN D'EXPERTISE ET DE RECHERCHE SUR LES AGENTS MICROBIENS - CEERAM CENTRE NATIONAL DE LA RECHERCHE SCIENTIFIQUE
<120> PROCÉDÉ DE GENOTYPAGE DE STAPHYLOCOCUS AUREUS
<130> PN000050WO
<150> FR10-54238
   <151> 2010-06-01
<160> 48
<170> PatentIn version 3.5
<210> 1
   <211> 18
   <212> DNA
   <213> artificial sequence
<220>
   <223> amorce anti-sens du loci VNTR sa0122
<400> 1
   gcaccaaaag aggaagac 18
<210> 2
   <211> 18
   <212> DNA
   <213> artificial sequence
<220>
   <223> amorce sens du loci VNTR Sa0122
<400> 2
   cagcagtagt gccgtttg 18
<210> 3
   <211> 25
   <212> DNA
   <213> artificial sequence
<220>
   <223> amorce anti-sens du loci VNTR Sa0311
<400> 3
   aaatgatatt ttcgcaaaat ttatt 25
<210> 4
   <211> 22
   <212> DNA
   <213> artificial sequence
<220>
   <223> amorce sens du loci VNTR Sa0311
<400> 4
   gtatcaacaa gtgatagcat ca 22
<210> 5
   <211> 20
   <212> DNA
   <213> artificial sequence
<220>
   <223> amorce anti-sens du loci VNTR Sa0387
<400> 5
   cattccaaac ataccatcac 20
<210> 6
   <211> 20
   <212> DNA
   <213> artificial sequence
<220>
   <223> amorce sens du loci VNTR Sa0387
<400> 6
   caaagtaata ggcactacaa 20
<210> 7
   <211> 20
   <212> DNA
   <213> artificial sequence
<220>
   <223> amorce anti-sens du loci VNTR Sa0550
<400> 7
   aacttgatcg acaccagagc 20
<210> 8
   <211> 21
   <212> DNA
   <213> artificial sequence
<220>
   <223> amorce sens du loci VNTR Sa0550
<400> 8
   gtgacagatg taagacttag a 21
<210> 9
   <211> 20
   <212> DNA
   <213> artificial sequence
<220>
   <223> amorce anti-sens du loci VNTR Sa0684
<400> 9
   caacaagttg ttcagcctgc 20
<210> 10
   <211> 21
   <212> DNA
   <213> artificial sequence
<220>
   <223> amorce sens du loci VNTR Sa0684
<400> 10
   aggtattgga agtgaaacag c 21
<210> 11
   <211> 20
   <212> DNA
   <213> artificial sequence
<220>
   <223> amorce anti-sens du loci VNTR Sa0964
<400> 11
   ccaacctgtt aatccgatgt 20
<210> 12
   <211> 21
   <212> DNA
   <213> artificial sequence
<220>
   <223> amorce sens du loci VNTR Sa0964
<400> 12
   atcccagatt atccaataca a 21
<210> 13
   <211> 24
   <212> DNA
   <213> artificial sequence
<220>
   <223> amorce anti-sens du loci VNTR Sa1097
<400> 13
   gcaacttctt aaaacaaaat attg 24
<210> 14
   <211> 23
   <212> DNA
   <213> artificial sequence
<220>
   <223> amorce sens du loci VNTR Sa1097
<400> 14
   gaattattgt tatcgccatt gtc 23
<210> 15
   <211> 20
   <212> DNA
   <213> artificial sequence
<220>
   <223> amorce anti-sens du loci VNTR Sa1194
<400> 15
   ggtgctaaag tcgatgtaat 20
<210> 16
   <211> 20
   <212> DNA
   <213> artificial sequence
<220>
   <223> amorce sens du loci VNTR Sa1194
<400> 16
   ctgtgtcggt aggttacatt 20
<210> 17
   <211> 20
   <212> DNA
   <213> artificial sequence
<220>
   <223> amorce anti-sens du loci VNTR Sa1729
<400> 17
   gaccatgcac tacgtgttac 20
<210> 18
   <211> 21
   <212> DNA
   <213> artificial sequence
<220>
   <223> amorce sens du loci VNTR Sa1729
<400> 18
   gtctcgaatc acttaacaac g 21
<210> 19
   <211> 21
   <212> DNA
   <213> artificial sequence
<220>
   <223> amorce anti-sens du loci VNTR Sa1866
<400> 19
   gcttgttggt tcagctttag g 21
<210> 20
   <211> 20
   <212> DNA
   <213> artificial sequence
<220>
   <223> amorce sens du loci VNTR Sa1866
<400> 20
   gctttacgtg taataacacc 20
<210> 21
   <211> 20
   <212> DNA
   <213> artificial sequence
<220>
   <223> amorce anti-sens du loci VNTR Sa0266
<400> 21
   cttccgattg ttcgatgctt 20
<210> 22
   <211> 20
   <212> DNA
   <213> artificial sequence
<220>
   <223> amorce sens du loci VNTR Sa0266
<400> 22
   ttggatatga agcgagacca 20
<210> 23
   <211> 19
   <212> DNA
   <213> artificial sequence
<220>
   <223> amorce anti-sens du loci VNTR Sa0704
<400> 23
   cgatcgcacg atatggtgg 19
<210> 24
   <211> 20
   <212> DNA
   <213> artificial sequence
<220>
   <223> amorce sens du loci VNTR Sa0704
<400> 24
   aagagtgtgt agggaatggc 20
<210> 25
   <211> 20
   <212> DNA
   <213> artificial sequence
<220>
   <223> amorce anti-sens du loci VNTR Sa1132
<400> 25
   tgggaggaat taatcatgtc 20
<210> 26
   <211> 20
   <212> DNA
   <213> artificial sequence
<220>
   <223> amorce sens du loci VNTR Sa1132
<400> 26
   ctagttcaag ctagatcagg 20
<210> 27
   <211> 19
   <212> DNA
   <213> artificial sequence
<220>
   <223> amorce anti-sens du loci VNTR Sa1291
<400> 27
   gtgttgctct tgaatcatc 19
<210> 28
   <211> 20
   <212> DNA
   <213> artificial sequence
<220>
   <223> amorce sens du loci VNTR Sa1291
<400> 28
   gtcaagacac agatattgct 20
<210> 29
   <211> 21
   <212> DNA
   <213> artificial sequence
<220>
   <223> amorce anti-sens du loci VNTR Sa2039
<400> 29
   cataaattca atgtcctagg c 21
<210> 30
   <211> 20
   <212> DNA
   <213> artificial sequence
<220>
   <223> amorce sens du loci VNTR Sa2039
<400> 30
   tatttcgttc taccccaact 20
<210> 31
   <211> 26
   <212> DNA
   <213> artificial sequence
<220>
   <223> amorce anti-sens du loci VNTR Sa2511
<400> 31
   aaggtcaaga atatttaaaa tcaatt 26
<210> 32
   <211> 22
   <212> DNA
   <213> artificial sequence
<220>
   <223> amorce sens du loci VNTR Sa2511
<400> 32
   ggcaaaatgc acatgaaaca ct 22
<210> 33
   <211> 331
   <212> DNA
   <213> artificial sequence
<220>
   <223> amplicon obtenu par amplification du VNTR Sa01223 avec les amorces SEQ ID NO: 1 et SEQ ID NO: 2 pour la souche Staphylococcus aureus Mu50
<400> 33
   cagcagtagt gccgtttgct tttgcaatgt catttactgt atcaccaggt ttaacgacat 60
   gtactccgtt gccgtcttct ttaccaggtt tgttgccgtc ttctttacca ggcttgttgc 120
   cgtcttcttt accaggcttg ttgttgtctt ctttaccagg cttgttgccg tcttctttgc 180
   caggtttgtt gttgtcttct ttaccaggct tgttgccgtc ttctttacca ggttttttgt 240
   tgtcttcttt accaggcttg ttgccgtctt ctttgccagg tttgttgccg tcttctttac 300
   caggtttttt gttgtcttcc tcttttggtg c 331
<210> 34
   <211> 316
   <212> DNA
   <213> artificial sequence
<220>
   <223> amplicon obtenu par amplification du VNTR Sa0311 avec les amorces SEQ ID NO: 3 et SEQ ID NO: 4 pour la souche Staphylococcus aureus Mu50
<400> 34
   gtatcaacaa gtgatagcat caaatggaga taaattataa aggtatatta taagtatttt 60
   agggttagag cccgagacat aaattaatat agtaggacct acagtgttat aatggcgggc 120
   ccccaacaca aagaatttcg aaaagaaatt ctacaggtaa tgcaagttgg cggggcccca 180
   acacagagaa attcgaaaag aaattctaca ggcaatgcaa gttggcgggg ccccaacaca 240
   gagaatttcg aaaagaaatt ctacaggtaa tgcaagttgg ggaaggacag gaataaattt 300
   tgcgaaaata tcattt 316
<210> 35
   <211> 255
   <212> DNA
   <213> artificial sequence
<220>
   <223> amplicon obtenu par amplification du VNTR Sa0387 avec les amorces SEQ ID NO: 5 et SEQ ID NO: 6 pour la souche Staphylococcus aureus Mu50
<400> 35
   caaagtaata ggcactacaa cgcttattgg cggggcccca acaaagaagc tgacgaaaag 60
   tcagcttaca ataatgtgca agttggggat gggccccaac atagagaaat tgggtccgta 120
   atttctacag acaatgcaag ttggcggggc cccaacaaat ttaagataca atgcgtaata 180
   ttaatatgtt attattatga taatttacag aattatatga aaaatgaatg aggatgtgat 240
   ggtatgtttg gaatg 255
<210> 36
   <211> 847
   <212> DNA
   <213> artificial sequence
<220>
   <223> amplicon obtenu par amplification du VNTR Sa0550 avec les amorces SEQ ID NO: 7 et SEQ ID NO: 8 pour la souche Staphylococcus aureus Mu50
<400> 36
   gtgacagatg taagacttag aaaaatacaa acagatggac gaatgaaagc actcgtttcc     60
   attacattag atgaagcttt cgtaattcat gatttacgtg taattgaagg aaactctggc    120
   ttgttcgttg caatgccaag taaacgtaca ccagatggtg aattccgcga catcgcgcat    180
   cctattaatt cagatatgag acaagaaatt caagatgcag tgatgaaagt atatgatgaa    240
   acagatgaag tagtaccaga taaaaacgct acatcagaag attcagaaga agcttaatca    300
   attttatatt tagcgatgta atacatttgc aataagttga tttgatactg tcgataaagc    360
   ataaagcttt gtcggcagtt tttttagttt gtattaatgt ttttttagtt tgtattaatg    420
   tttttttagt ttgtattaat gtttttttag tttgtattaa tgttttttta tttttaatga    480
   aaggctaata aatatatacg ttaacagatt atgatgatat gaaaattatt gattcattgc    540
   tgataaaaat tgcgtttgaa tgatgctcgt atttttgaag taagaaaaaa gttgttttta    600
   aaattacaac gaattaaaaa caatgccttt tatatgttga aagagtattg cagattaaat    660
   tataataatg acgaagtgta aaatttaatg ggggttaatg ttcatgcgaa gacacgcgat    720
   aattttggca gcaggtaaag gcacaagaat gaaatctaaa aagtataaag tgctacacga    780
   ggttgctggg aaacctatgg tcgaacatgt attggaaagt gtgaaaggct ctggtgtcga    840
   tcaagtt                                                              847
<210> 37
   <211> 1000
   <212> DNA
   <213> artificial sequence
<220>
   <223> amplicon obtenu par amplification du VNTR Sa0684 avec les amorces SEQ ID NO: 9 et SEQ ID NO: 10 pour la souche Staphylococcus aureus Mu50
<400> 37
   aggtattgga agtgaaacag cagatgtctt acttgtttat atattcggac gtattgaatt       60
   tattccagat agctatacaa gaaaaatata tgataaatta ggatatgaaa acactaaaaa      120
   ttatgatcaa ttaaaaaaag tagtcacatt accaaatcat tttacaaatc aagatgctaa      180
   tgaatttcat gctctgttag atgtatttgg taaacattac tttagagaca aagatataaa      240
   gaattatgat tttttagaac cttactttaa aaagtaaacg ctgtgaagtt agatagatga      300
   gtttatatga aatataaaaa ataatttact attttctttt agtatgtgga cttatataat      360
   aaatagaagc atataaagaa aaaaacagtt gtttgtttgt gcagcaactg cataaaagcc      420
   tctaatcact aaaggtgaag aggaacgcag ttggatgcta aggcacaact gcataaatcc      480
   ctctaatcgc taaagcgaaa agtgggatta aaaaggagat gtgataatgt gaagaaatcg      540
   ttaattgctt ttattttgat ttttatgctt gtcctgagtg gctgtggtat gaaagataat      600
   gataaacaag gtagcgatga taatggctcg tctaaatcgc cgtaccatag aattgtttcg      660
   ttaatgccta gtaatactga aattttatat gaattaggat taggtaaata catagttggt      720
   gtttcaacgg ttgatgatta tccaaaagat gtgaaagagg gtaagaaaca atttgatgct      780
   ttgaatctaa ataaagagga acttttaaag gcaaagccag atctaattct tgcgcatgag      840
   tcgcaaaagg caactgctaa taaagtattg tcatcattag agaaacaagg catcaaagta      900
   gtgtatgtta aagatgcaca atcaattgat gaaacataca acacatttaa gcaaattggg      960
   aaattaacgc atcatgataa gcaggctgaa caacttgttg                           1000
<210> 38
   <211> 597
   <212> DNA
   <213> artificial sequence
<220>
   <223> amplicon obtenu par amplification du VNTR Sa0964 avec les amorces SEQ ID NO: 11 et SEQ ID NO: 12 pour la souche Staphylococcus aureus Mu50
<400> 38
   atcccagatt atccaataca aaatgagcta acaagtagca taagaaaagc cgcagcaaac 60
   atcggcgaca aagagttaac acatatgtgg agtggacaaa gcccgcgact agcaacaacg 120
   catcccgcca acaccatcat gtccaatata atcaatcaaa ttaatcaaat catgcaatat 180
   aaataatcga ccgcaatcca caaaagcaca agcactccca aacattattt tagtgcttgc 240
   catttttgtg gattgcgttt ctattttacc aatctagtca aacgaaatta tcaaactaaa 300
   gttcgctgaa atgattatag tcaaacgaaa acatcaaact aaagttcgct gaaatgatta 360
   tgatcaaacg aaattatcga gctgaaggtc accgaaatga ttataattaa acgaaattat 420
   cgagctaaag ttcactgaaa tgattataat caaacgaaaa catcaagctg aagatcgccg 480
   aaagatttta atcaagcgaa aacatcaaat taaagttcgc tgaaatgatt atgataaaag 540
   ttatatggta tgatgacatt ggtgatatat atgataaaca tcggattaac aggttgg 597
<210> 39
   <211> 196
   <212> DNA
   <213> artificial sequence
<220>
   <223> amplicon obtenu par amplification du VNTR Sa1097 avec les amorces SEQ ID NO: 13 et SEQ ID NO: 14 pour la souche Staphylococcus aureus Mu50
<400> 39
   gaattattgt tatcgccatt gtctggatta tcagggttgt tagggttgtc agggttattt 60
   ggttcatctg ggttgttagg gttatcagga ttgttagggt tatcaggatt gttagggtta 120
   tcaggattat ctgggttatt aggttggtca tcgttggcaa aatggatatc ttcaatattt 180
   tgttttaaga agttgc 196
<210> 40
   <211> 874
   <212> DNA
   <213> artificial sequence
<220>
   <223> amplicon obtenu par amplification du VNTR Sa1194 avec les amorces SEQ ID NO: 15 et SEQ ID NO: 16 pour la souche Staphylococcus aureus Mu50
<400> 40
   ctgtgtcggt aggttacatt aattaccagc caattccgag attcccaggc atgtctcgtg 60
   acattgcatt agaagtagat caaaatattc cagcagctga tttattatca acgattcatg 120
   cacacggtgg caatatatta aaagatacac ttgtctttga tgtatatcag ggcgaacatt 180
   tagaaaaagg taaaaaatca attgcaatac gtttaaatta tttagacaca gaagaaacat 240
   tgacagatga gcgcgtttca aaagtgcaag cggaaattga agcagcatta attgaacaag 300
   gtgctgttat tagataatga tttaaacccc atgtataagg atatctgaag tagattgata 360
   tccttaacat ggggttttat ttttggattc accaattagg tttcgattca tttaaaaatc 420
   aaagaggcac agcggaatac agatgatgct tctcacaact gcatataaag tcatacttat 480
   gagcaaagca aattagtatg acttacctaa taggatttcc tattaggatc caatttatct 540
   tgattttata aatcatagag gcacagtgga ttacagatga tgcttcgcat cactgcatat 600
   aaagtcatac ttatgagcaa agcaaattag tatgacttac ctaataggat cacctattag 660
   gatccaattc attttataaa tcaaagagga acagcggaat acagatgatg cttcgcacaa 720
   ctgcataaaa gcctctaatg attaaaaatc aaagaggctt taaaattttt tgggcttttt 780
   cacgattttt aaaatgcttt tttgaaatgg tatctaaacg tgaaagaccg tattttttta 840
   taatttttgc ggcaattaca tcgactttag cacc 874
<210> 41
   <211> 797
   <212> DNA
   <213> artificial sequence
<220>
   <223> amplicon obtenu par amplification du VNTR Sa1729 avec les amorces SEQ ID NO: 17 et SEQ ID NO: 18 pour la souche Staphylococcus aureus Mu50
<400> 41
   gtctcgaatc acttaacaac gaataaaata taatctaata tcattttcat tcatgaaaaa        60
   gcgggaatgg aatagaaatg ctaaagaacc attaacggtt tattatgtaa tggttcttcc       120
   acattagcca ccactattat gtacttaaaa ataggaatac ataattagat tcatgcatag       180
   ggagtgggac agaaatgata ttttaacaaa attaaattcg ttatccccaa ctggcattgc       240
   ctgtagaatt tctttacgaa attctctgtg ttggggcccc atccccaact tgcacattat       300
   tgaaagctga cttttggtca gcttctatgt tgggggcccc gtccccaact tccattgcct       360
   gttgaaatta gggttccaat ttctctgtgt tggggccccg ccaacttcca ttgcctgttg       420
   aaattggggt tccaatttct ctatgttggg gccccgtccc caacttccat tgcctgtaga       480
   atttctttac gaaattctct atgttggggc cccgccaata taacattgta gagcctagga       540
   cattgtgatg tcccagactc tatcatcatg aattattctc atcaaaaact gtctttcgtc       600
   attttcgacg ttgaaacttc aaataagtaa tttattgttg ccattgttta tacaacaaaa       660
   tttaattgac cttcattttt gaacacatcg tcaattgttg caccaccaag acacacatca       720
   ccttgataaa aaacaactgc ttgtccaggt gtgattgctc ttactggctc agcaaaagta       780
   acacgtagtg catggtc                                                       797
<210> 42
   <211> 933
   <212> DNA
   <213> artificial sequence
<220>
   <223> amplicon obtenu par amplification du VNTR Sa1866 avec les amorces SEQ ID NO: 19 et SEQ ID NO: 20 pour la souche Staphylococcus aureus Mu50
<400> 42
   gctttacgtg taataacacc attttcaaat ttagcttctt tttgtttaac agtgtcttta        60
   ttatctatat taaccgtctc tgtctctgat acatcattct ttttagtaac taattgagga       120
   atttctttca agtcattttt agcaactggt ttttctgcaa ataacgcttc agttaattga       180
   ctctcttttg aacctggtgt taatttagct tgcttctgtt ttgcagcgtt tgctaatctt       240
   tcagcttgtg actctggttt tgatacttct acctttgctg cacttgcatc tttagttgat       300
   gtgtcactgt ctttctctgc ttgtgcttct tttttagctt cttgaacctc ttgtgcctct       360
   tgtgatgtat cacttaaatt atttgcactt gcttcttctt ttatcgctgc ttgttgtgct 420
   ttcaatgcca ctgcttttgg ttcttcattt gatacagctg cacttttatc tgtttctgct 480
   tgtgcttctt ttttagcttc ttgaatctct tgtgcctctt gtgacgtgtc acttaaatta 540
   tttgcacttg cttcttcttt tattgctgct tgttgtactt ttaatgccga tgcttttgac 600
   tcttcatttg atacagccac acttttatcc gcttctgctt gtgcttctct tttagcttct 660
   tgaatctctt gtgcttcttg tgatgtatca cttaaattat ttgcacttgc ttcttctttt 720
   atcgctgctt gttgtgcctt taatgccgct tgctcatttt tagatttgtt taaaaatcct 780
   tcaacacgtt cttttgtata ggcaaccgtt tcttcaagtt gcgtttttct ttcttcaaac 840
   ttttgcgaca gttcttgttc tttgactttt aaatcatctg ctttttgata aactttattt 900
   ttaaaataca aacctaaagc tgaaccaaca agc 933
<210> 43
   <211> 630
   <212> DNA
   <213> artificial sequence
<220>
   <223> amplicon obtenu par amplification du VNTR Sa0266 avec les amorces SEQ ID NO: 21 et SEQ ID NO: 22 pour la souche Staphylococcus aureus Mu50
<400> 43
   ttggatatga agcgagacca agattcaaca agccaagtga aacaaatgca tacaacgtaa 60
   cgacaaatca agatggcaca gtatcatacg gagctcgccc aacacaaaac aagccaagtg 120
   aaacaaacgc atataacgta acaacacatg caaatggtca agtatcatac ggtgctcgcc 180
   caacacaaaa aaagccaagc aaaacaaatg catacaacgt aacaacacat gcaaatggtc 240
   aagtatcata tggcgctcgc ccgacacaaa aaaagccaag caaaacaaat gcatataacg 300
   taacaacaca tgcaaatggt caagtatcat acggagctcg cccgacatac aagaagccaa 360
   gcgaaacaaa tgcatacaac gtaacaacac atgcaaatgg tcaagtatca tatggcgctc 420
   gcccgacaca aaaaaagcca agcgaaacaa acgcatataa cgtaacaaca catgcagatg 480
   gtactgcgac atatgggcct agagtaacaa aataaattta taactctatc caaagacata 540
   cagtcaatac aaagaattat gtatctatac aacagtaatc atgcattcta tgatgcttct 600
   aactgaatta aagcatcgaa caatcggaag 630
<210> 44
   <211> 303
   <212> DNA
   <213> artificial sequence
<220>
   <223> amplicon obtenu par amplification du VNTR Sa0704 avec les amorces SEQ ID NO: 23 et SEQ ID NO: 24 pour la souche Staphylococcus aureus Mu50
<400> 44
   aagagtgtgt agggaatggc gttgtataaa ttgtattaga agaacttcta acgcatctct      60
   gtggttaaaa gagatgaagg gaacgacagt ttaattaaaa ctgcataaga acttctagct     120
   tttctctttc gttcaaagag aagcagctgt tcgcagttta atcaaaacca cataaagctt     180
   ttaactttac tctttgattt aaagagtgac aaatgtttac agtttaatta aaactgcata     240
   agaacttcta gcttttctct ttcgttcaaa gagaagttct aataccacca tatcgtgcga     300
   tcg                                                                   303
<210> 45
   <211> 884
   <212> DNA
   <213> artificial sequence
<220>
   <223> amplicon obtenu par amplification du VNTR Sa1132 avec les amorces SEQ ID NO: 25 et SEQ ID NO: 26 pour la souche Staphylococcus aureus Mu50
<400> 45
   ctagttcaag ctagatcagg ttcaaagggt ttgagggcaa gcctcatctc agtataaaac      60
   acccctagtg tgtgcgattt atttaattaa ttatactgta agacgtttgt aaacttatgt     120
   caataggttg tcttcatgaa atttcgttta attcgattta aaatttataa tattagcatt     180
   ggatttaaat tgaagatgta gtaggaatgt tagtaattaa agatataaaa atatgtgaca     240
   tgtaataaga ttgagctgat aaatgaagag ggatacttat caatcatacc tctttaacaa     300
   cagtgaagaa cccgtgcata atggcttacg aattatagtt tgtagggtaa aagagggata     360
   catgcgccga gcacatgcaa aaaagcccct aacaactaaa agttgtaagg taaaagaggg     420
   atacatgcgc cgagcacatg caaaaaagcc cctaacaact aaaagttgta aggtaaaaga     480
   gggatacatg caccgagcgc aagcaaaaaa gcccctaaca actaaaggtt gtaaggaaga     540
   agagggatac atgcaccgag cacatgcata aaatccccta acaactaaaa gttgtaaggt     600
   aaaagaggga tacatgcgcc gagcacatgc ataaaatccc ctaacaacta aaagttgtaa     660
   ggaagaagag gaacacatgc gccgagcaca tgcataaaag cccctaacaa ctaaaagttg     720
   taaggggatt taaattaatt tagtgtatct tggatatctt gttttgtttg attaatatct     780
   tctgtttttt cttctttttt atcttttaat ttttcttcaa cttctttagc tttttctgct     840
   gcttttttat tttgattttc attagacatg attaattcct ccca                       884
<210> 46
   <211> 870
   <212> DNA
   <213> artificial sequence
<220>
   <223> amplicon obtenu par amplification du VNTR Sa1291 avec les amorces SEQ ID NO: 27 et SEQ ID NO: 28 pour la souche Staphylococcus aureus Mu50
<400> 46
   gtcaagacac agatattgct aatacagtag cgttcttagc atcagacaaa gcaaaatata      60
   ttacaggtca aacaatccat gtaaatggtg gaatgtacat gtaatatatt tgagctaaag     120
   ctcattgacg cagtggttga ctggtcatcc aatggagaat tgtctgacct agtcaacttt     180
   gcgggggaaa ttctaagcaa cctagataag gttccagaat ttctccctaa gaaacactaa     240
   tcaataaatt gataagaaga ttagaggaac gcttgagcta aagctcaaga taaagacgca     300
   gaaatcaata aattgataac tgggatagag gtacgcctga gctaaagctc aagataaaga     360
   cgcagtaatc aataaattga taactgggat agaggtacac ctgagctaaa gctcatgcat     420
   aagaaacact aatcaataaa ttgataagtg tttctaaaat ttctacttgt tttttagaat     480
   ttaaaatggg aaaatataat agtctatgta taggcatttt taaaggaggt gaatcgacgt     540
   ggaaaatttc gataaagtaa aagatatcat cgttgaccgt ttaggtgtag acgctgataa     600
   agtaactgaa gatgcatctt tcaaagatga tttaggcgct gactcacttg atatcgctga     660
   attagtaatg gaattagaag acgagtttgg tactgaaatt cctgatgaag aagctgaaaa     720
   aatcaacact gttggtgatg ctgttaaatt tattaacagt cttgaaaaat aataaatctt     780
   acatctgggt cgtcagtatt gtcgactcag tttttttctt taattatcaa tagttttaac      840
   gtaaaattaa agatgattca agagcaacac                                      870
<210> 47
   <211> 275
   <212> DNA
   <213> artificial sequence
<220>
   <223> amplicon obtenu par amplification du VNTR Sa2039 avec les amorces SEQ ID NO: 29 et SEQ ID NO: 30 pour la souche Staphylococcus aureus Mu50
<400> 47
   tatttcgttc taccccaact tgcattgtct gtagaatttc ctttcgaaat tctctttgtt      60
   tggggcccca ccccaacttg cacattattg taagctgaca gaaagtcagc ttctatgttg     120
   gggccccgcc aacttgcaca ttattgtaag ctgacagaaa gtcagcttct atgttggggc     180
   cccactagaa ttgaaaaaag cttgttacaa gcgtattttc tttcagtcaa ctactgccaa     240
   tataacattg tagtgcctag gacattgaat ttatg                                275
<210> 48
   <211> 370
   <212> DNA
   <213> artificial sequence
<220>
   <223> amplicon obtenu par amplification du VNTR Sa2511 avec les amorces SEQ ID NO: 31 et SEQ ID NO: 32 pour la souche Staphylococcus aureus Mu50
<400> 48
   ggcaaaatgc acatgaaaca ctaattaagc tttaattttg agtaatatag aagatattaa      60
   aagcagcagt aagatgtttt ctaattgaaa attatcttac tgctgctttt taagaattac     120
   tgtgtcagtc tattttcggt tcgcctgcgc ttttgcgcat gcataaaagc ccctaacaac     180
   ctgaggtcac tgcgcttcgg ttcgcctgcg cttttgcgca tgcataaaag cccctaacaa     240
   cctgaggtca ctgcgcttcg gttcgcctgc gcttttgcgc atgcataaaa gcccctaaca     300
   accaaaggtt gtaaggggct ttaaatgtca gatagtattt tttaaattga ttttaaatat     360
   tcttgacctt                                                            370

## Description

La présente invention concerne un procédé de typage automatisé et discriminant de *Staphylococcus aureus.* Un autre but de la présente invention se rapporte à un kit pour la mise en oeuvre du procédé susmentionné.

Le genre *Staphylococcus* comprend plus de 50 espèces et sous-espèces mais la plus fréquente en pathologie humaine est représentée par *Staphylococcus aureus.* Cette bactérie commensale se retrouve dans diverses niches écologiques comme la peau ou la plupart des muqueuses chez les mammifères à sang chaud. Chez l'homme, *S. aureus* se loge principalement au niveau des fosses nasales et près de 30% de la population comprendrait des porteurs sains.

La contamination se produit le plus souvent au contact de la peau car les infections surviennent généralement lors d'une rupture de la barrière cutanéo-muqueuse. Cependant, un affaiblissement du système immunitaire peut également conduire à des infections très diverses en brisant le fragile équilibre qui existe entre la bactérie et son hôte. En effet, une fois dans le sang, la bactérie peut facilement coloniser l'ensemble des organes vitaux et induire des pathologies mortelles comme des endocardites, des chocs toxiques, des septicémies ou encore des pneumonies nécrotiques.

L'augmentation drastique de souches résistantes aux antibiotiques constitue un trait majeur de la virulence de *S*. *aureus.* En effet, *S. aureus* a montré une incroyable capacité d'adaptation en modulant son spectre de résistance aux antibiotiques selon leurs utilisations. L'utilisation non raisonnée d'antibiotiques dans les hôpitaux a induit une sélection de souches multirésistantes à la plupart des dérivés méticilline ainsi qu'à certains glycopeptides. Les cliniciens se retrouvent alors dans une situation d'impasse thérapeutique engendrée par l'acquisition de cette multirésistance. La virulence de cette bactérie couplée à sa multirésistance aux antibiotiques assure à *Staphylococcus aureus* le deuxième rang des pathogènes nosocomiaux avec plus de 30% des infections nosocomiales recensées.

*Staphylococcus aureus* est également pathogène pour de nombreux mammifères, notamment des animaux d'élevage, bovins, ovins et caprins. En France, *S. aureus* est l'une des principales causes de ce type d'infection avec près de 30% des mammites diagnostiquées. Cette pathologie, dont l'enjeu est majeur en élevage laitier, touche près d'un quart des ruminants allaitants et se manifeste par une réduction de la quantité et de qualité du lait et dans les cas les plus graves par une nécrose de la glande mammaire.

*Staphylococcus aureus* représente un enjeu majeur pour de nombreux secteurs : clinique, vétérinaire et agro-alimentaire. Les professionnels des secteurs concernés doivent mettre en place les mesures adéquates afin d'identifier les sources de contamination ainsi que les modes et vecteurs de contamination. L'engagement vers l'emploi de ce type de procédures préventives est d'autant plus important que les outils utilisés pour éradiquer ce pathogène sont parfois défaillants. Pour répondre à ce besoin, il est nécessaire de savoir distinguer les différentes souches au sein de l'espèce considérée. Le génotypage permet de caractériser la variabilité génétique de ces populations et d'identifier alors les relations de parenté entre les souches qui les composent.

### ETAT DE LA TECHNIQUE

L'utilisation récente de techniques de typage moléculaire faisant appel aux méthodes d'empreintes génétiques (« DNA fingerprinting ») a augmenté considérablement la puissance des méthodes de typage bactérien qui, traditionnellement, se fondaient sur l'utilisation de marqueurs phénotypiques peu discriminants et à la reproductibilité médiocre. Avant l'ère du séquençage en masse des génomes bactériens, les techniques moléculaires de génotypage reposaient sur la détection par hybridation de séquences d'insertion (IS-RFLP : polymorphisme de longueur des fragments de restriction de séquence d'insertion), d'amplification PCR (amplification en chaîne par polymérase), d'amplification au hasard RAPD (Amplification au hasard d'ADN polymorphe), AFLP (polymorphisme de longueur de fragments amplifiés) ou de différences à des sites de restriction. La PFGE (Electrophorèse sur gel en champ pulsé) repose sur l'analyse des profils de macrorestriction de l'ADN total par électrophorèse en champ pulsé. Le résultat est un profil de restriction définissant un pulsotype caractérisant l'isolat bactérien étudié. La PFGE demeure la technique de typage standard pour de nombreuses espèces bactériennes dont *S. aureus.* Une collection de référence, composée de 98 isolats MRSA, a été établie par un groupe de travail de l'IUMS (Union internationale des sociétés de microbiologie - « International Union of Microbiology Societies ») dans le cadre d'un projet de lutte contre les infections nosocomiales. Ce projet appelé Harmonisation des mesures de résistance aux antibiotiques, Procédé de typage des organismes et moyens de les utiliser et autres outils afin d'accroître l'efficacité du contrôle des infections nocosomiales (« Harmonisation of Antibiotic Resistance measurement, Methods of typing Organisms and ways of using these and other tools to increase the effectiveness of Nosocomial Infection control ») a donné son nom à la collection HARMONY et a conduit au développement d'un protocole de PFGE harmonisé et standardisé. Cette méthode a été utilisée avec succès dans le cadre de nombreuses enquêtes épidémiologiques ainsi que pour des études macro-épidémiologiques de grande ampleur. Cependant, la PFGE est en cours d'abandon dans les laboratoires de biologie moléculaire eu égard à ses nombreux inconvénients techniques et pratiques : la technique est longue, couteuse et difficile à mettre en oeuvre; les inconvénients majeurs étant le manque de reproductibilité inter-laboratoire, le défaut évident de standardisation des résultats obtenus et l'instabilité des marqueurs étudiés qui indexent rapidement l'accumulation des variations génétiques.

Au cours de la dernière décennie, la disponibilité croissante des séquences génomiques complètes de nombreuses bactéries pathogènes a permis de développer de nouvelles techniques. L'une d'entres elles, le typage par séquençage multi-locus MLST (« Multi Locus Sequence Typing ») est désormais utilisée comme technique de premier choix pour des études globales et pour l'établissement de relations phylogénétiques solides entre isolats de *Staphylococcus aureus.* Cette méthode est basée sur le polymorphisme de séquences de sept fragments internes de gènes de ménage qui présentent des variations intra-spécifiques. Chaque locus d'intérêt est séquencé puis l'allèle identifié est associé à un caractère numérique. Ainsi, pour chaque souche, le profil allèlique représentant le génotype («sequence type» (ST)) est défini par un code numérique. Le typage par MLST permet donc facilement l'échange des données produites dans différents laboratoires. Ces échanges sont facilités par l'existence d'une base publique pour les données de typage de *S. aureus* par MLST (http://saureus.mlst.net). Le MLST montre que l'évolution de *Staphylococcus aureus* se fait par mutation ponctuelle et non par recombinaison : la population de *Staphylococcus aureus* est donc clonale. L'importance du génome dit accessoire est mise en exergue par le MLST. En effet, cette méthode reposant sur l'analyse des gènes de ménage, constituant le génome dit central, ne corrèle pas les ST avec l'acquisition de la résistance, la virulence, ou la propension à coloniser un hôte spécifique, caractéristiques alors associées au génome accessoire.

Cependant, compte tenu du faible taux de mutation des gènes de ménage, le MLST demeure une technique de typage peu discriminante et donc inusitée dans des contextes épidémiques. De plus, l'application en routine du typage par MLST dans les laboratoires de biologie moléculaire n'est pas encore envisageable. En effet, le typage d'un isolat comprend l'amplification et le séquençage de sept fragments PCR (sens et anti-sens pour chaque locus étudié aboutissant à l'obtention de 14 séquences), cette méthode reste donc encore très coûteuse, fastidieuse et longue (le résultat de typage MLST d'un isolat est assuré en 12 heures avec un séquenceur 4-capillaires).

Avec la possibilité d'analyser les génomes bactériens *in silico,* la MLVA ou l'Analyse de plusieurs locus VNTR (Répétition en tandem polymorphe (« Multiple Locus VNTR Analysis ») a également pu émerger.

Les VNTRs sont des répétitions en tandem présentant un polymorphisme intra-espèce du nombre de répétitions justifiant ainsi leur utilisation comme marqueurs moléculaires.

Les répétitions en tandem sont des séquences génomiques, présentes dans les régions codantes ou intergéniques, caractérisées par la répétition d'un motif nucléotidique élémentaire. Ces séquences sont également souvent appelées microsatellites lorsque le motif répété comprend moins de 9 paires de bases et minisatellites lorsque le motif répété comprend 9 paires de base et plus.

Le génotypage par MLVA repose sur l'amplification par PCR de ces régions ciblées, dispersées sur le génome bactérien, à l'aide d'amorces spécifiques des régions flanquantes, et sur la détermination des tailles des amplicons par électrophorèse permettant d'évaluer le nombre de répétitions à un locus donné. Les motifs répétés peuvent compter de une à plus de cent paires de bases et la taille des amplicons varie de quelques dizaines à plus de mille nucléotides. La longueur des unités répétées étant connue, ces tailles reflètent le nombre de séquences répétées dans les régions amplifiées. Cette méthode d'empreintes génétiques augmente considérablement, pour certaines espèces, la puissance des méthodes de génotypage bactérien. Le résultat du typage est un code numérique incluant le nombre de motifs à chaque locus. Ce type de génotypes numériques, dont l'interprétation est facilitée, est parfaitement adapté aux études comparatives intra et inter laboratoire menées dans le cadre d'un suivi ou d'une enquête épidémiologique.

Plusieurs publications traitent de la thématique MLVA *Staphylococcus aureus* au sens le plus général du terme. Parmi ces articles, quatre semblent devoir faire référence en la matière : 1) Pourcel et al., J. Clin. Microbiol. 2009,47(10):3121-8 ; 2) Schouls et al., Plos One. 2009,4(4):e5082 ; 3) Hardy et al., Microbiology. 2004 Dec, 150 (Pt12):4045-52 et 4) Sabat et al., J. Clin. Microbiol. 2003 Apr,41(4): 1051-66.

Le protocole développé par Sabat et collaborateurs ne constitue pas un MLVA mais une MLVF : empreinte de plusieurs loci VNTRs (« Multiple Locus VNTR Fingerprinting »). Cette technique repose sur l'analyse et la comparaison de profils obtenus par l'analyse concomitante de six à sept VNTRs. Cette méthode, bien que discriminante car reposant sur l'analyse du polymorphisme de plusieurs VNTRs, ne présente pas les mêmes commodités que le MLVA (incapacité de coder le génotype, difficulté d'échange de résultats, ...).

Les travaux de Hardy et collaborateurs ont décrit les premiers véritables protocoles de typage par MLVA pour *Staphylococcus aureus.* Le protocole MLVA défini par Hardy repose sur l'analyse en simplex sur agarose de sept VNTRs (nommés SIRU pour Staphylococcal Interspersed Repeat Unit par analogie avec l'acronyme MIRU utilisé pour certaines répétitions en tandem de *Mycobacterium tuberculosis.* Quatre études ont validé ce protocole sur une collection de souches cliniques et une étude a montré ses limites sur une collection de souches animales.

Un protocole MLVA à 14 marqueurs, dont 10 primordiaux, a été publié par l'équipe GPMS de l'IGM (Pourcel et al., 2009). Ce protocole a été validé sur plus de 500 souches dont la collection HARMONY. Les travaux de Hardy et collaborateurs ainsi que Pourcel et collaborateurs montrent que le MLVA satisfait tous les critères de performance relatifs à une excellente technique de typage : stabilité des marqueurs utilisés, typabilité excellente (supérieure à 98%), reproductibilité parfaite et pouvoir de discrimination élevé (similaire à celui du SBT à six loci). Cependant, dans ces travaux, l'analyse des tailles des loci a été réalisée par électrophorèse sur gel d'agarose. Il paraît difficile d'envisager d'entreprendre le typage systématique par MLVA des nouveaux isolats identifiés dans un contexte de suivi épidémiologique en utilisant le même support.

Les recherches de Schouls et collaborateurs constituent un progrès significatif dans le typage de *S. aureus* par MLVA (Schouls et al., 2009). En effet, l'article est le premier et le seul à ce jour à publier un protocole MLVA validé sur électrophorèse capillaire ABI3730 en deux multiplexes de quatre VNTRs. La technique s'est avérée performante sur la collection de souches testées (2525 dont 100 souches animales issues de porcs) mais la comparaison avec le MLST n'a pas été effectuée. Cependant, les amorces utilisées ne s'apparient pas toutes correctement avec les 14 génomes séquencés de *Staphylococcus aureus* et le nombre de marqueurs analysés est encore insuffisant pour permettre un typage à la résolution convenable.

La nécessité du développement d'une nouvelle méthode de typage moléculaire s'impose donc pour permettre la réalisation d'un typage bactérien de *Staphylococcus aureus* discriminant à haut-débit, de routine et à moindre coût.

### RESUME DE L'INVENTION

La présente invention a pour but un nouveau procédé de typage automatisé et discriminant de *Staphylococcus aureus.* Un autre but de la présente invention est de fournir des kits (ou trousses) pour la mise en oeuvre du procédé susmentionné.

A cet effet, l'invention concerne un procédé de génotypage de l'espèce *Staphylococcus aureus* par MLVA comprenant les étapes consistant à :
(i) réaliser une première co-amplification de dix loci VNTRs par PCR multiplexe dans un premier milieu réactionnel comprenant un échantillon d'ADN issu d'un isolat de Staphylococcus aureus le premier milieu réactionnel comprenant les couples d'amorces respectivement anti-sens/sens suivants : SEQ ID NO :1 /SEQ ID NO :2 ; SEQ ID NO :3/ SEQ ID NO :4 ; SEQ ID NO :5/ SEQ ID NO :6 ; SEQ ID NO :7/ SEQ ID NO :8 ; SEQ ID NO :9/ SEQ ID NO : 10 ; SEQ ID NO : 11/ SEQ ID NO :12 ; SEQ ID NO 13/ SEQ ID NO 14 ; SEQ ID NO :15/ SEQ ID NO :16; SEQ ID NO : 17/ SEQ ID NO :18; SEQ ID NO : 19/ SEQ ID NO :20, afin de former plusieurs amplicons pour chacun des dix loci VNTRs,
   puis une seconde co-amplification d'au moins six loci VNTRs supplémentaires par PCR multiplexe dans un second milieu réactionnel, chacun des premier et second milieux réactionnels comprenant un échantillon d'ADN issu du même isolat de Staphylococcus aureus, le second milieu réactionnel comprenant les couples d'amorces respectivement anti-sens/sens suivants : SEQ ID NO : 21/ SEQ ID NO : 22; SEQ ID NO : 23/ SEQ ID NO : 24; SEQ ID NO : 25/ SEQ ID NO : 26; SEQ ID NO : 27/ SEQ ID NO : 28; SEQ ID NO : 29/ SEQ ID NO : 30 et SEQ ID NO : 31/ SEQ ID NO : 32,
(ii) analyser lesdits amplicons par électrophorèse capillaire afin d'obtenir un profil électrophorétique dudit échantillon, et
(iii) attribuer un code numérique MLVA audit profil électrophorétique.

La présente invention repose sur la technique de typage MLVA combinée à une PCR multiplexe apte à co-amplifier au moins dix loci VNTRs en une seule fois, en une seule étape. En effet, la présente invention représente le premier procédé de typage du pathogène *Staphylococcus aureus* par une seule réaction de PCR, et est le seul procédé ayant dépassé le multiplexage de plus de dix VNTRs pour un typage par MLVA. Jusqu'à présent, il était possible de co-amplifier maximum quatre loci VNTRs mais pas au moins dix. Le procédé selon l'invention représente donc une réelle avancée technologique.

Par définition, le terme de "PCR multiplexe" désigne une mise au point de la technique PCR autorisant l'amplification, en une seule réaction, de plusieurs segments d'ADN distincts. Le premier avantage de cette adaptation technique est la réduction du coût et la diminution du temps de réalisation et d'analyse des résultats. La réalisation d'une PCR multiplexe doit répondre à de nombreuses contraintes techniques.

Ainsi, un des intérêts du procédé de l'invention réside dans l'identification d'un produit PCR multiplexe, comme l'allèle de VNTRs connus. Il n'est pas judicieux d'analyser le résultat de la PCR multiplexe sous forme d'un profil de bandes, souvent peu reproductibles et dont la standardisation est hasardeuse. Le procédé selon l'invention a donc été conçu pour une application du typage avec analyse des fragments amplifiés par électrophorèse capillaire et validés sur les appareils de type Applied Biosystems Genetic Analyser 3100-Avant, 3130, 3130xl et 3500.

En outre, la technique MVLA employée avec la PCR multiplexe permet un typage précis de la souche identifiée et convient également parfaitement à des études comparatives fines entre souches bactériennes dans un délai très court (moins de quatre heures).

Par conséquent, le présent demandeur a ainsi mis au point une méthode permettant un génotypage du pathogène *Staphylococcus aureus* à un coût suffisamment réduit pour envisager sa large utilisation dans les laboratoires de microbiologie en test de premier rang.

En particulier, le code numérique MLVA de l'étape (iii) est obtenu automatiquement par informatique, à partir de logiciel d'analyse de fragments ou de génotypage.

Le profil électrophorétique obtenu peut être analysé avec plusieurs logiciels différents disponibles en libre accès (Peak Scanner^{®}, CLC Sequence Viewer^{®},...). Cependant, le traitement des données électrophorétiques ainsi que l'automatisation des résultats de typage sont possibles par l'utilisation du logiciel GeneMapper^{®} (Applied Biosystems). En particulier, ce logiciel peut être associé à trois fichiers mis au point par le demandeur afin d'obtenir le code numérique MLVA automatiquement. Ces fichiers sont ainsi à importer sous GeneMapper^{®}, qui décrivent les différents paramètres à ajuster et les données à interpréter afin qu'un utilisateur non initié puisse obtenir simplement le code MLVA de la souche typée avec pour chaque locus un indice indiquant le degré de confiance attribué à la valeur associée (nombre de motifs répétés pour le VNTR considéré). Ainsi, le fichier nommé «Méthode d'analyse.xml » permet le traitement des données analysées selon des critères définis et les fichiers nommés « MLVA management A.txt » et « MLVA management B.txt » compilent l'ensemble des données recueillies sur les différents allèles associés à chaque marqueur défini par une gamme de taille et de couleur. Ces fichiers sont décrits plus explicitement par la suite.

Par conséquent, le présent procédé présente l'avantage de permettre un typage automatique, rapide, au pouvoir de discrimination élevé, reproductible et à haut-débit de *S*. *aureus* dans le cadre d'une application en typage de routine. Or, au vu de l'état de la technique actuel, il n'était pas évident pour un homme du métier de mettre au point une technique permettant l'obtention de l'ensemble de ces avantages.

Avantageusement, le milieu réactionnel de l'étape (i) comprend : au moins dix amorces sens marquées et au moins dix amorces anti-sens, formant au moins dix couples d'amorces sens/anti-sens pour chacun desquels correspond un locus VNTR spécifique et différent pour chacun des dix couples d'amorces, chacune des dix amorces sens s'hybridant en amont de son locus respectif et chacune des dix amorces anti-sens s'hybridant en aval de son locus respectif.

Par définition les amorces sens et anti-sens sont des séquences oligonucléotidiques capables de s'hybrider de façon spécifique, grâce à la complémentarité des bases, respectivement sur le brin d'ADN anti-sens i.e. le brin non codant transcrit et sur le brin d'ADN sens i.e. le brin codant non transcrit.

Selon une caractéristique avantageuse de l'invention, les au moins dix amorces sens sont marquées par un fluorophore en 5'.

Préférentiellement, le fluorophore est choisi parmi : 6-FAM, NED, PET™ (Applied Biosystems) ou VIC.

Chacune des amorces sens peut donc être marquée avec un fluorophore défini par un spectre d'excitation unique et permet alors d'associer un VNTR donné à une gamme de couleur ou de motifs lors de la visualisation des profils électrophorétiques.

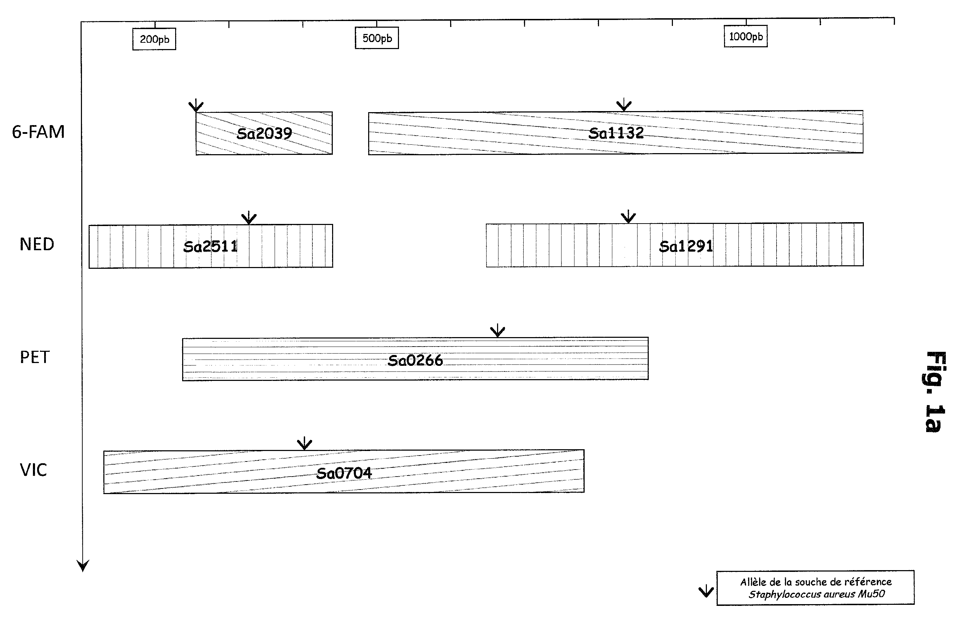

De plus, les amorces sens et anti-sens peuvent être élaborées de telle sorte que chaque VNTR soit associé à une gamme de taille connue. Ainsi, un plan de multiplexage calibré par les deux dimensions spectrale, assurée par le marquage des amplicons, et spatiale, garanti par le choix pertinent des amorces, permet d'identifier d'une manière unique chaque VNTR (voir le plan de multiplexage Figure 1a).

De manière préférée, le milieu réactionnel comprend en outre au moins dix amorces sens supplémentaires non marquées.

En particulier, le ratio : amorces sens non marquées sur amorces anti-sens est de l'ordre de 2/3 et le ratio : amorces sens marquées sur amorces anti-sens est de l'ordre de 1/3.

Selon un mode de réalisation préféré, est réalisée à l'étape (i), une première co-amplification de dix loci VNTRs par PCR multiplexe dans un premier milieu réactionnel, et une seconde co-amplification d'au moins six loci VNTRs supplémentaires par PCR multiplexe dans un second milieu réactionnel, chacun des premier et second milieux réactionnels comprenant un échantillon d'ADN issu du même isolat de *Staphylacoccus aureus.*

Le premier milieu réactionnel comprend les couples d'amorces respectivement anti-sens/sens suivants : SEQ ID NO :1 /SEQ ID NO :2 ; SEQ ID NO :3/ SEQ ID NO :4 ; SEQ ID NO :5/ SEQ ID NO :6 ; SEQ ID NO :7/ SEQ ID NO :8 ; SEQ ID NO :9/ SEQ ID NO :10 ; SEQ ID NO :11/ SEQ ID NO :12 ; SEQ ID NO :13/ SEQ ID NO :14 ; SEQ ID NO :15/ SEQ ID NO :16; SEQ ID NO : 17/ SEQ ID NO :18; SEQ ID NO : 19/ SEQ ID NO :20.

Le second milieu réactionnel comprend les couples d'amorces respectivement anti-sens/sens suivants : SEQ ID NO : 21/ SEQ ID NO : 22; SEQ ID NO : 23/ SEQ ID NO : 24; SEQ ID NO : 25/ SEQ ID NO : 26; SEQ ID NO : 27/ SEQ ID NO : 28; SEQ ID NO : 29/ SEQ ID NO : 30 et SEQ ID NO : 31/ SEQ ID NO : 32.

Ce mode de réalisation de la présente invention permet ainsi l'analyse de seize VNTRs en deux étapes successives comprenant dans un premier temps l'analyse d'un panel de dix VNTRs (nommé Panel 1) et dans un second temps l'analyse d'un panel de six VNTRs (nommé Panel 2). Par conséquent, la présente invention peut décrire un schéma MLVA à seize VNTRs composé de ces deux panels complémentaires. Le panel 1 permet d'assigner les isolats typés aux groupes phylogénétiques majeurs. Ce panel est intéressant dans le cadre d'études macro-épidémiologiques. En effet il permet d'avoir, en une seule PCR, une idée générale sur la diversité génétique d'une population bactérienne assurant un index de diversité supérieur à celui du MLST et identique à celui du champ pulsé tandis que le panel 2 permet d'assurer un caractère résolutif très supérieur au typage. La combinaison des deux panels garantit une résolution extrêmement fine permettant d'effectuer une étude micro-épidémiologique dans un cadre spatio-temporel restreint.

Pour ce mode de réalisation, le second milieu réactionnel de l'étape (i) peut comprendre en outre six autres amorces sens marquées et six autres amorces anti-sens correspondant aux six loci VNTRs supplémentaires.

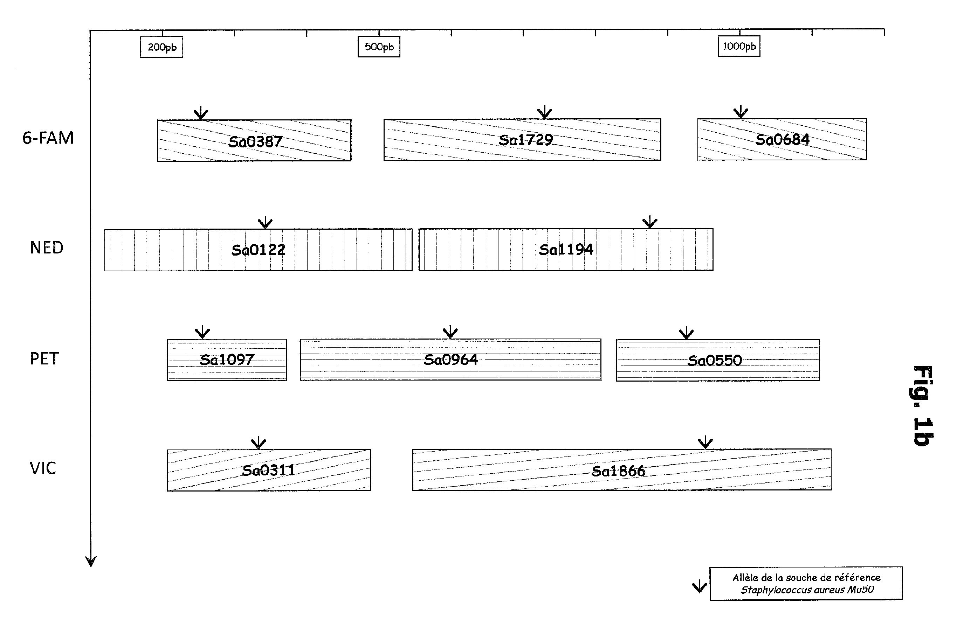

Ces amorces sens et anti-sens ont été élaborées de telle sorte que chaque VNTR soit associé à une gamme de taille connue. Ainsi, un plan de multiplexage calibré par les deux dimensions spectrale, assurée par le marquage des amplicons, et spatiale, garanti par le choix pertinent des amorces, permet d'identifier d'une manière unique chaque VNTR (voir le plan de multiplexage Figure 1b).

De manière avantageuse, les séquences des amorces sont comprises entre 15 pb et 30 pb, de préférence entre 18pb et 25pb, ne comprenant pas plus de six, de préférence pas plus de quatre répétitions mononucléotidiques et avec des pourcentages en GC de préférence compris entre 20 et 80, de préférence encore entre 40 et 60.

De préférence, la différence des températures de désappariement des amorces est inférieure à 15 °C, de préférence inférieure à 10 °C, de préférence encore inférieure à 8°C.

La présente invention se rapporte également à un kit de génotypage de *Staphylococcus aureus* pour la mise en oeuvre du procédé de génotypage tel que décrit ci-dessus, comprenant :
- un premier milieu réactionnel comportant dix amorces sens marquées et dix amorces anti-sens, formant dix couples d'amorces sens/ anti-sens pour chacun desquels correspond un locus VNTR spécifique et différent pour chacun des dix couples d'amorces, chacune des dix amorces sens s'hybridant en amont de son locus respectif et chacune des dix amorces anti-sens s'hybridant en aval de son locus respectif, les couples d'amorces respectivement anti-sens/sens étant les suivants : SEQ ID NO :1 /SEQ ID NO :2 ; SEQ ID NO :3/ SEQ ID NO :4 ; SEQ ID NO :5/ SEQ ID NO :6 ; SEQ ID NO :7/ SEQ ID NO :8 ; SEQ ID NO :9/ SEQ ID NO : 10 ; SEQ ID NO :11/ SEQ ID NO :12 ; SEQ ID NO :13/ SEQ ID NO :14 ; SEQ ID NO :151 SEQ ID NO 16; SEQ ID NO : 17/ SEQ ID NO :18; SEQ ID NO : 19/ SEQ ID NO :20,
- un second milieu réactionnel comprenant les couples d'amorces respectivement anti-sens/sens suivants : SEQ ID NO : 21/ SEQ ID NO : 22; SEQ ID NO : 23/ SEQ ID NO : 24; SEQ ID NO : 25/ SEQ ID NO : 26; SEQ ID NO : 27/ SEQ ID NO : 28; SEQ ID NO : 29/ SEQ ID NO : 30 et SEQ ID NO : 31/ SEQ ID NO : 32.
- un mix d'amplification,
- un témoin positif et un témoin négatif.

Par conséquent, les amorces utilisées présentent, pour chaque panel, la triple particularité i) de garantir l'unicité d'un VNTR sur un profil électrophorétique par la caractérisation du vecteur taille/couleur, ii) de permettre leur association dans une seule réaction de PCR (la formation de structures en dimères ou hairpin entre les amorces a été recherchée) et iii) de s'hybrider au niveau de zones conservées chez l'espèce *Staphylococcus aureus*.

### DESCRIPTION DETAILLEE

L'invention sera mieux comprise et d'autres buts, détails, caractéristiques et avantages de celle-ci apparaîtront plus clairement à la lecture de tests expérimentaux, en référence aux figures annexées dans lesquelles :
- la Figure 1a représente le plan de multiplexage selon la présente invention pour 10 loci VNTRs (Panel 1) et la Figure 1b pour les 6 VNTRs supplémentaires (Panel 2);
- la Figure 2 représente (sur deux pages 2.1 et 2.2) un dendrogramme réalisé selon l'UPGMA [Unweighted Pair Group Method with Arithmetic mean] (méthode de groupe de paires hiérarchiques non pondérées avec l'agrégation (cluster) de l'analyse de la moyenne arithmétique) présentant l'agrégation des isolats de la collection HARMONY (89 isolats) par le protocole de typage selon l'invention (16 loci VNTRs) ;
- la Figure 3 représente (sur deux pages 3.1 et 3.2) un dendrogramme réalisé selon l'UPGMA présentant l'agrégation des isolats de la collection HARMONY (89 isolats) par le protocole de typage selon l'invention (Panel 1) ;
- la Figure 4 représente (sur deux pages 4.1 et 4.2) un dendrogramme réalisé selon l'UPGMA présentant l'agrégation des isolats de la collection HARMONY (89 isolats) par le protocole de typage selon l'invention (Panel 2) ;
- la Figure 5 représente (sur deux pages 5.1 et 5.2) un dendrogramme réalisé selon l'UPGMA présentant l'agrégation des isolats de la collection HARMONY (89 isolats) par le protocole de typage de Pourcel et col. (10 loci VNTRs par MVLA et sans PCR multiplexe) (art antérieur) ;
- la Figure 6 représente (sur deux pages 6.1 et 6.2) un dendrogramme réalisé selon l'UPGMA présentant l'agrégation des isolats de la collection HARMONY (89 isolats) par le protocole de typage de Schouls et col. (8 loci VNTRs par MLVA en deux PCR) (art antérieur) ;
- la Figure 7 représente (sur deux pages 7.1 et 7.2) un dendrogramme réalisé selon l'UPGMA présentant l'agrégation des isolats de la collection HARMONY (89 isolats) par le protocole de typage MLST7 (technique de l'art antérieur) ;
- la Figure 8 représente trois tableaux 10a, 10b et 10e correspondant aux résultats pour une analyse de reproductibilité ;
- et les Figures 9, 10 et 11 représentent les fichiers mis au point par le présent demandeur pour obtenir le code MVLA automatiquement, c'est-à-dire :

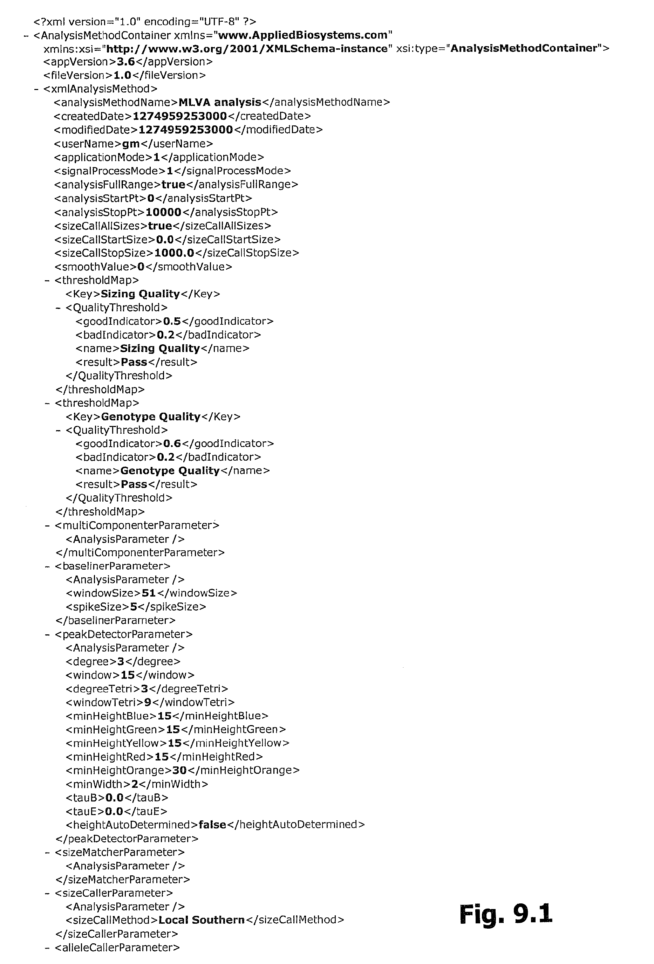

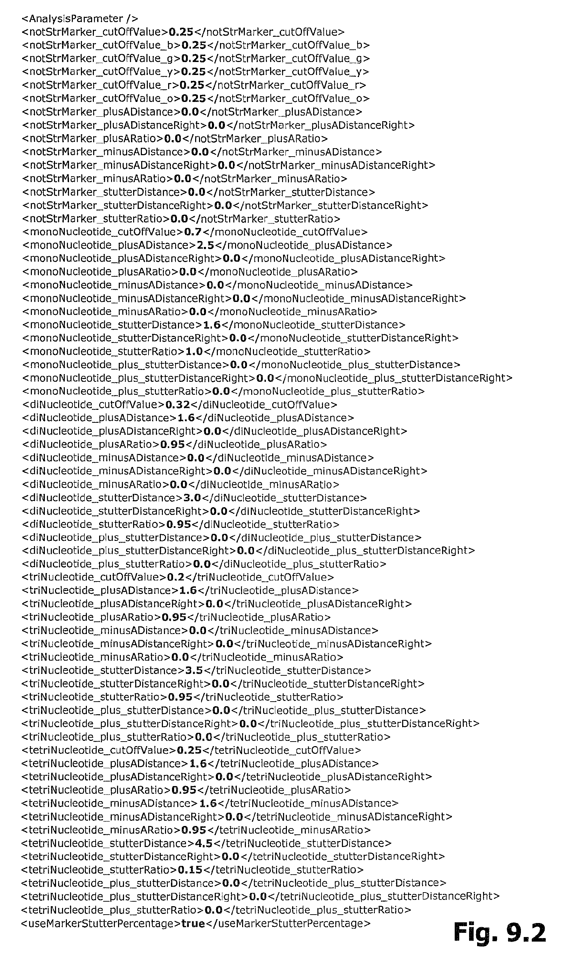

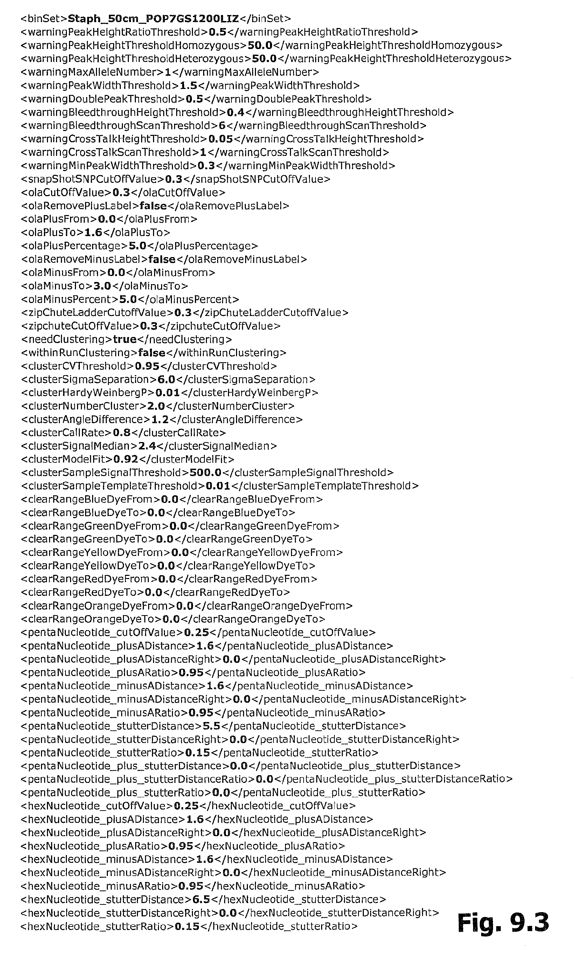

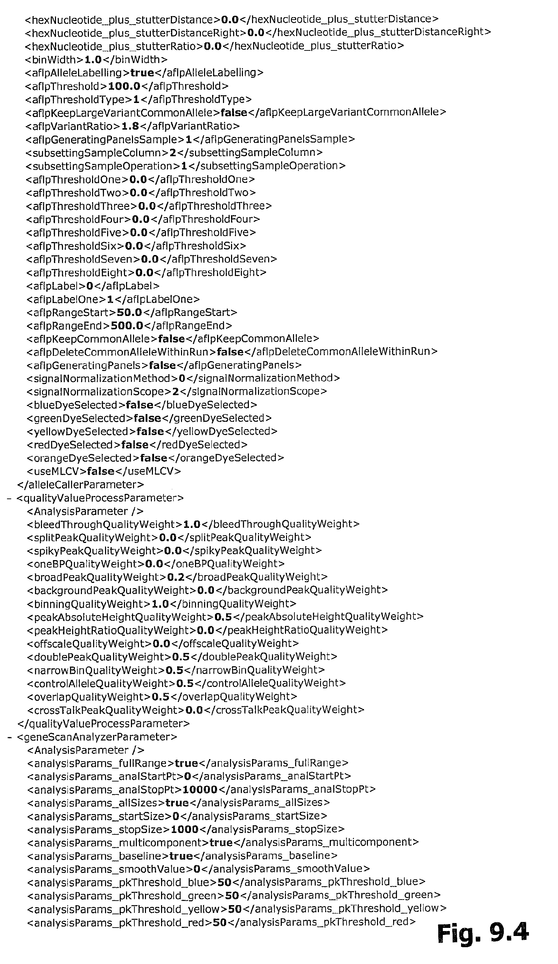

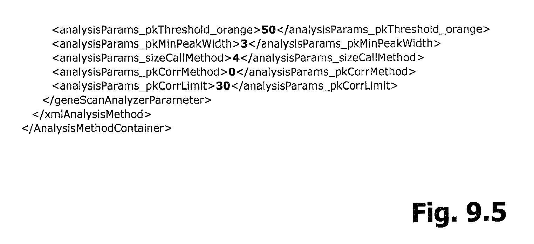

   Figure 9 (sur cinq pages 5.1 à 5.5) pour la méthode d'analyse.xml,

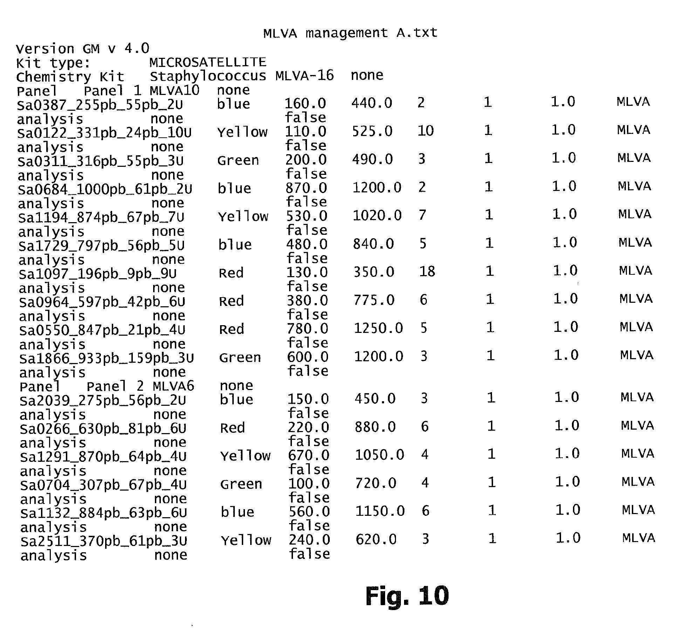

   Figure 10 pour MLVA Management A.txt,

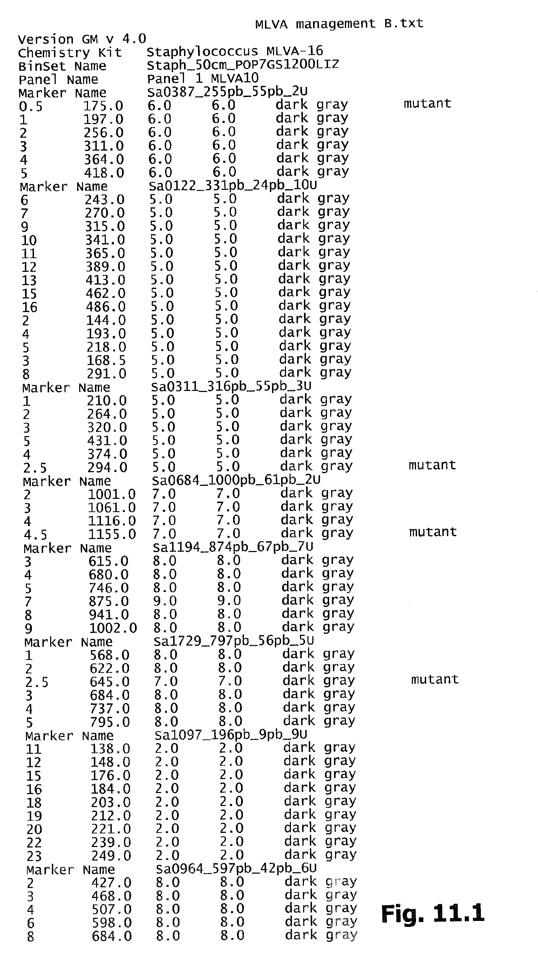

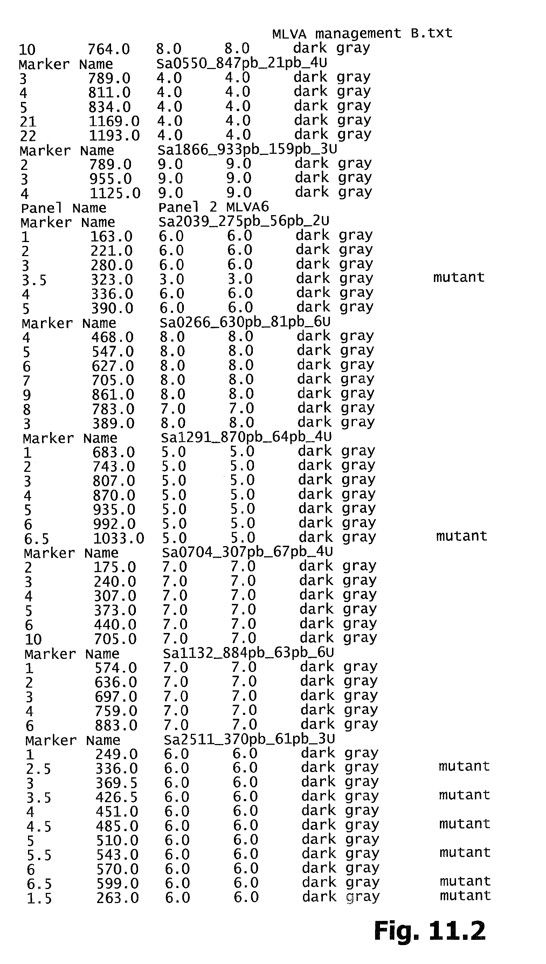

   Figure 11 (sur deux pages 11.1 et 11.2) pour MLVA Management B.txt.

### A) Détails expérimentaux

La section suivante décrit les matériels et méthodes utilisés pour le génotypage de *Staphylococcus aureus* par le procédé de la présente invention. Elle sera décomposée en cinq parties : 1) Choix et sélection des VNTRs, 2) Définition des panels, 3) Elaboration des amorces et construction du plan de multiplexage, 4) PCR multiplexe et analyse des fragments et 5) Obtention du code MLVA.

### 1) Choix et sélection des VNTRs

La présence des 16 VNTRs choisis, dans les génomes des 14 souches de *S. aureus* séquencés, a été recherchée à l'aide de la base de données : http://minisatellites.u-psud.fr. Dans la section « Bacteria » de la partie « Access the microbial tandem repeats database main page », chaque souche séquencée est choisie successivement et chacun des VNTR est recherché par la taille du motif nucléotidique élémentaire répété. Les 14 souches présentent les 16 VNTRs.

Finalement, le présent demandeur a choisi une combinaison unique de seize marqueurs, décrit dans le *Tableau 1* ci dessous, pour le typage par MLVA de *Staphylococcus aureus.*

### 2) Définition des panels

Le protocole de typage par MLVA décrit se décline sous deux formes représentées par deux panels garants de deux niveaux de discrimination différents. Le premier panel est constitué de dix VNTRs alors que le second en comprend six. Le panel 1 a été constitué de telle sorte qu'il soit assez discriminant pour être utilisé seul et le plus congruent possible avec le protocole à seize VNTRs.

**Tableau 1 : Caractéristiques des VNTRs utilisés dans la présente invention (Les caractéristiques associées à chaque VNTR sont propres à la souche Staphylococcus aureus Mu50)**

| Nom simplifié du VNTR | Panel | Taille du motif répété (pb) | Nombre de répétitions | Taille théorique de l'amplicon (pb) | Position sur le génome (kb) | Type de séquences présentant le VNTR |
|---|---|---|---|---|---|---|
| Sa0122 | 1 | 24 | 10 | 331 | 122 | protein A |
| Sa0311 | 1 | 55 | 3 | 316 | 311 | Intergénique |
| Sa0387 | 1 | 55 | 2 | 255 | 387 | Intergénique |
| Sa0550 | 1 | 21 | 5 | 847 | 550 | Intergénique |
| Sa0684 | 1 | 66 | 2 | 1000 | 684 | Intergénique |
| Sa0964 | 1 | 43 | 6 | 597 | 964 | Intergénique |
| Sa1097 | 1 | 9 | 18 | 196 | 1097 | cystéine protease |
| Sa1194 | 1 | 67 | 7 | 874 | 1194 | Intergénique |
| Sa1729 | 1 | 56 | 5 | 721 | 1729 | Intergénique |
| Sa1866 | 1 | 159 | 3 | 933 | 1866 | hypothetical protein |
| Sa0266 | 2 | 81 | 6 | 630 | 266 | staphylocoagulase |
| Sa0704 | 2 | 67 | 4 | 303 | 704 | Intergénique |
| Sa1132 | 2 | 63 | 6 | 884 | 1132 | Intergénique |
| Sa1291 | 2 | 64 | 4 | 870 | 1291 | Intergénique |
| Sa2039 | 2 | 56 | 3 | 275 | 2039 | Intergénique |
| Sa2511 | 2 | 61 | 3 | 370 | 2511 | Intergénique |

### 3) Elaboration des amorces et construction du plan de multiplexage

Une fois les loci d'intérêt choisis, l'originalité développée par la présente invention réside, selon un mode de réalisation préféré, dans la capacité à co-amplifier par PCR multiplexe respectivement 10 VNTRs dans le cas du panel 1 et 6 VNTRs dans le cas du panel 2. Pour cela, la pertinence dans le choix des amorces est-importante. Comme évoqué précédemment dans la description de l'invention, le choix de la séquence des amorces utilisées a été réalisé pour remplir trois objectifs :
i) les amorces doivent assurer l'unicité d'identification d'un amplicon, issu de chaque multiplexe, par la visualisation d'un plan de multiplexage.
ii) les amorces doivent permettre la co-amplification efficace des marqueurs respectifs des panels 1 et 2
iii) les amorces doivent être choisies au niveau de séquences conservées de diverses souches de l'espèce *Staphylococcus aureus* afin que le protocole de typage présente une typabilité proche de 100%.

Aucune hiérarchie ou chronologie n'est établie entre ces objectifs pour effectuer le choix des amorces. L'ensemble de ces paramètres est à prendre en compte et les trois critères de décision s'imbriquent. Cependant, par souci de clarté, chacun d'entre eux sera détaillé indépendamment.
i) Le typage de la collection HARMONY, représentative de la diversité des souches cliniques MRSA, et de plus de 1000 autres isolats cliniques donne une estimation de la gamme d'allèle pour chaque VNTR. Il est ainsi raisonnable de disposer et d'arranger les gammes d'allèles afin d'établir deux plans de multiplexage pour chaque panel. Ces plans, illustrés par un schéma en 2D (spatiale et spectrale), représentent l'agencement des différents amplicons issus des deux multiplexes. La première dimension est imposée par l'attribution d'un des 4 fluorophores suivants : 6-FAM, NED, PET™, VIC. La présente invention étant développée sur les plateformes d'analyse Applied Biosystems Genetic Analyser 3100-Avant, 3130, 3130x1 et 3500, les fluorophores sont choisis chez Applied Biosystems. La seconde dimension est définie par une gamme de taille propre à chaque VNTR. Le croisement de ces deux dimensions permet d'attribuer une position spectrale et spatiale unique pour chaque VNTR. Pour une couleur donnée, chaque gamme de taille est séparée par au moins la taille d'une unité répétée. De plus, les amorces des VNTRs de type microsatellite sont systématiquement choisies de telle sorte que les amplicons soient de petite taille. Après avoir considéré l'ensemble de ces paramètres, les plans de multiplexage ont été construits comme illustré par les Figures 1a et 1b.
ii) Une fois la configuration spatiale des VNTRs connue, des zones génomiques au sein desquelles les amorces peuvent être élaborées sont définies. Classiquement, les séquences des amorces doivent avantageusement répondre à plusieurs critères : séquence comprise entre 18pb et 25pb, %GC compris entre 40 et 60, ne comprenant pas plus de 4 répétitions mononucléotidiques. Ensuite, d'autres exigences sont spécifiques à la réalisation de la PCR multiplexe. Ainsi, afin que les 10 et 6 produits puissent respectivement être co-amplifiés au sein des panels 1 et 2, de manière efficace et homogène, en utilisant un même protocole PCR et *a fortiori* une même température d'hybridation, les amorces présentent de préférence une température de désappariement (Tm) voisine. Les inventeurs ont empiriquement imposé une différence maximale de 8°C entre les différents Tm. De plus, la co-amplification exigeant le rassemblement de tous les réactifs dans une seule réaction, il est important que les amorces restent neutres entre elles, c'est-à-dire qu'elles ne donnent pas d'hybridation croisée. La probabilité de formation de structures en dimères par les amorces a été testée par le logiciel Autodimer. Après importation d'un fichier fasta comprenant l'ensemble des séquences à valider, ce logiciel indique si des couples sont susceptibles de se former. Enfin, une queue nucléotidique de 7pb est ajoutée en position 5' de chaque amorce anti-sens afin de faciliter l'adénylation des produits PCR. En effet, l'ajout partiel d'une adénine en 3' des amplicons par la Taq polymérase entraine une difficulté d'analyse des profils électrophorétiques ; on appelle cet artefact le « pic +A ». L'étape finale du choix des amorces est la démonstration de leur spécificité. Chaque séquence est donc soumise à l'algorithme BLAST et recherchée dans tous les génomes séquencés.
iii) Une des caractéristiques principales d'une bonne technique de typage est la typabilité, c'est-à-dire la capacité à attribuer un génotype à tous les isolats typés. Autrement dit, l'amplification des 16 VNTRs doit être possible pour l'ensemble de l'espèce *Staphylococcus aureus* et un code MLVA complet (composé de 16 valeurs) doit être attribué. Classiquement une technique de typage dont la typabilité est supérieure à 97% est considérée comme excellente. Deux raisons peuvent provoquer l'absence d'amplification : l'absence du VNTR et la non hybridation des amorces. Concernant la présence des VNTRs pour l'ensemble des souches séquencées, celle-ci a été préalablement vérifiée sur les 14 génomes séquencés. Cependant, ces 14 souches ne sont surement pas représentatives de la diversité de l'espèce ; il est donc impossible de prédire la conservation d'une séquence génétique pour l'ensemble de l'espèce. Cependant, la comparaison de ces génomes permet d'identifier des zones conservées et de désigner les amorces au niveau de celles-ci.

Les amorces utilisées pour la présente invention répondant aux trois critères i), ii) et iii) ont été élaborées ; leurs caractéristiques sont détaillées dans les *Tableau 2a et 2b* ci-après.

**Tableau 2a : Caractéristiques des amorces utilisées dans la présente invention (Les amorces, décrites utilisées pour la co-amplification des 10 VNTRs du panel 1)**

| Nom du VNTR amplifiés | SEQ ID NO | Type d'amorce (Le fluorophore utilisé pour le marquage est indiqué entre parenthèses) | Séquence |
|---|---|---|---|
| Sa0122 | SEQ ID NO: 1 | Anti-sens non marquée | GCACCAAAAGAGGAAGAC |
| | SEQ ID NO: 2 | Sens non marquée | CAGCAGTAGTGCCGTTTG |
| | SEQ ID NO: 2 | Sens marquée (NED) | NED-CAGCAGTAGTGCCGTTTG |
| Sa0311 | SEQ ID NO: 3 | Anti-sens non marquée | AAATGATATTTTCGCAAAATTTATT |
| | SEQ ID NO: 4 | Sens non marquée | GTATCAACAAGTGATAGCATCA |
| | SEQ ID NO: 4 | Sens marquée (VIC) | VIC-GTATCAACAAGTGATAGCATCA |
| Sa0387 | SEQ ID NO: 5 | Anti-sens non marquée | CATTCCAAACATACCATCAC |
| | SEQ ID NO: 6 | Sens non marquée | CAAAGTAATAGGCACTACAA |
| | SEQ ID NO: 6 | Sens marquée (6FAM) | 6FAM-CAAAGTAATAGGCACTACAA |
| Sa0550 | SEQ ID NO: 7 | Anti-sens non marquée | AACTTGATCGACACCAGAGC |
| | SEQ ID NO: 8 | Sens non marquée | GTGACAGATGTAAGACTTAGA |
| | SEQ ID NO: 8 | Sens marquée (PET) | PET-GTGACAGATGTAAGACTTAGA |
| Sa0684 | SEQ ID NO: 9 | Anti-sens non marquée | CAACAAGTTGTTCAGCCTGC |
| | SEQ ID NO: 10 | Sens non marquée | AGGTATTGGAAGTGAAACAGC |
| | SEQ ID NO: 10 | Sens marquée (6FAM) | 6FAM-AGGTATTGGAAGTGAAACAGC |
| Sa0964 | SEQ ID NO: 11 | Anti-sens non marquée | CCAACCTGTTAATCCGATGT |
| | SEQ ID NO: 12 | Sens non marquée | ATCGCAGATTATCGAATACAA |
| | SEQ ID NO: 12 | Sens marquée (PET) | PET-ATCCCAGATTATCCAATACAA |
| Sa1097 | SEQ ID NO: 13 | Anti-sens non marquée | GCAACTTCTTAAAACAAAATATTG |
| | SEQ ID NO: 14 | Sens non marquée | GAATTATTGTTATCGCCATTGTC |
| | SEQ ID NO: 14 | Sens marquée (PET) | PET-GAATTATTGTTATCGCCATTGTC |
| Sa1194 | SEQ ID NO: 15 | Anti-sens non marquée | GGTGCTAAAGTCGATGTAAT |
| | SEQ ID NO: 16 | Sens non marquée | CTGTGTCGGTAGGTTACATT |
| | SEQ ID NO: 16 | Sens marquée (NED) | NED-CTGTGTCGGTAGGTTACATT |
| Sa1729 | SEQ ID NO: 17 | Anti-sens non marquée | GTGATGTGTGTCTTGGTGG |
| | SEQ ID NO: 18 | Sens non marquée | GTCTCGAATCACTTAACAACG |
| | SEQ ID NO: 18 | Sens marquée (6FAM) | 6FAM-GTCTCGAATCACTTAACAACG |
| Sa1866 | SEQ ID NO: 19 | Anti-sens non marquée | GCTTGTTGGTTCAGCTTTAGC |
| | SEQ ID NO: 20 | Sens non marquée | GCTTTACGTGTAATAACACC |
| | SEQ ID NO: 20 | Sens marquée (VIC) | VIC-GCTTTACGTGTAATAACACC |

**Tableau 2b : Caractéristiques des amorces utilisées dans la présence invention (Les amorces décrites utilisées pour la co-amplification des 6 VNTRs du panel 2)**

| Nom du VNTR amplifié | SEQ ID NO | Type d'amorce (Le fluorophore utilisé pour le marquage est indiqué entre parenthèses) | Séquence |
|---|---|---|---|
| Sa0266 | SEQ ID NO: 21 | Anti-sens non marquée | CTTCCGATTGTTCGATGCTT |
| | SEQ ID NO: 22 | Sens non marquée | TTGGATATGAAGCGAGACCA |
| | SEQ ID NO: 22 | Sens marquée (PET) | PET-TTGGATATGAAGCGAGACCA |
| Sa0704 | SEQ ID NO: 23 | Anti-sens non marquée | CGATCGCACGATATGGTGG |
| | SEQ ID NO: 24 | Sens non marquée | AAGAGTGTGTAGGGAATGGC |
| | SEQ ID NO: 24 | Sens marquée(VIC) | VIC-AAGAGTGTGTAGGGAATGGC |
| Sa1132 | SEQ ID NO: 25 | Anti-sens non marquée | TGGGAGGAATTAATCATGTC |
| | SEQ ID NO: 26 | Sens non marquée | CTAGTTCAAGCTAGATCAGG |
| | SEQ ID NO: 26 | Sens marquée (6FAM) | 6FAM-CTAGTTCAAGCTAGATCAGG |
| Sa1291 | SEQ ID NO: 27 | Anti-sens non marquée | GTGTTGCTCTTGAATCATC |
| | SEQ ID NO: 28 | Sens non marquée | GTCAAGACACAGATATTGCT |
| | SEQ ID NO: 28 | Sens marquée (NED) | NED-GTCAAGACACAGATATTGCT |
| Sa2039 | SEQ ID NO: 29 | Anti-sens non marquée | CATAAATTCAATGTCCTAGGC |
| | SEQ ID NO: 30 | Sens non marquée | TATTTCGTTCTACCCCAACT |
| | SEQ ID NO: 30 | Sens marquée (6FAM) | 6FAM-TATTTCGTTCTACCCCAACT |
| Sa251 1 | SEQ ID NO: 31 | Anti-sens non marquée | AAGGTCAAGAATATTTAAAATCAATT |
| | SEQ ID NO: 32 | Sens non marquée | GGCAAAATGCACATGAAACACT |
| | SEQ ID NO: 32 | Sens marquée (NED) | NED-GGCAAAATGCACATGAAACACT |

### 4) PCR multiplexe et analyse des fragments

Deux réactions de PCR permettent le typage d'un isolat de *Staphylococcus aureus*. Deux constituants participent à chacune de ces réactions : un mix d'amplification et un mix d'amorces.

Le mix d'amplification utilisé est le QIAGEN Multiplex PCR 2X Kit fourni par Qiagen.

Le mix d'amorces est constitué d'oligonucléotides marqués et non marqués obtenus chez Applied Biosystems et d'eau PPi. L'amplification d'un VNTR a utilisé ici la combinaison de 3 amorces : une amorce anti-sens, une amorce sens et une amorce sens marquée par un fluorophore (les séquences des amorces sens marquées et non-marquées sont identiques). L'équimolarité entre les amorces sens et anti-sens est assurée par l'établissement d'un ratio entre la concentration des amorces sens et de celle de l'amorce anti-sens. Dans les exemples selon la présente invention, les ratios sont : [amorces sens non marquées] / [amorces anti-sens] = 2/3 et [amorces sens marquées] / [amorces anti-sens] = 1/3. Chacun des triplets d'amorces utilisés pour l'amplification du VNTR associé est élaboré selon les ratios précédents.

Les concentrations finales nécessaires de chaque triplet ont été ajustées afin de permettre l'homogénéité d'intensité des pics lors de l'analyse des profils électrophorétiques. La concentration finale de l'amorce anti-sens, suffisante pour connaître les concentrations finales, est indiquée dans les *Tableau 3a et 3b* ci dessous.

**Tableau 3a : Concentration finale utilisée en amorce anti-sens pour la co-amplification des 10 VNTRs du panel 1**

| Nom simplifié du VNTR amplifié | Concentration finale en amorce anti-sens (en µM) |
|---|---|
| Sa0311,Sa0964 | 0,6 |
| Sa0387, Sa0550, Sa1097, Sa1866 | 0,3 |
| Sa0122, Sa0684, Sa1194 | 0,15 |
| Sa1729 | 0,05 |

**Tableau 3b : Concentration finale utilisée en amorce anti-sens pour la co-amplification des 6 VNTRs du panel 2**

| Nom simplifié du VNTR amplifié | Concentration finale en amorce anti-sens (en µM) |
|---|---|
| Sa2511 | 0,3 |
| Sal132, Sa1291, Sa2039 | 0,15 |
| Sa0266, Sa0704 | 0,05 |

A titre d'exemple, deux mix d'amorces (un pour la réalisation de chaque panel) pour 100 réactions, i.e. 100 typages, respectant les conditions ci dessus est décrit dans les *Tableau 4a et 4b.*

**Tableau 4a : Constitution d'un mix d'amorces pour 100 typages (Panel 1) Un volume d'eau de 865 µL est ajouté**

| Nom du VNTR | Nom de l'amorce | Volume d'amorce (à 20µM) à prélever |
|---|---|---|
| Sa0311, Sa0964 | Sa0311 R, Sa0964R | 45 |
| | Sa0311F, Sa0964F | 30 |
| | Sa0311F-VIC, Sa0964F-PET | 15 |
| Sa0387, Sa0550, Sa1097, Sa1866 | Sa0387R, Sa0550R, Sa1097R, Sa1866R | 22,5 |
| | Sa0387F, Sa0550F, Sa1097F, Sa1866F | 15 |
| | Sa0387F-6FAM, Sa0550F-PET, Sa1097F-PET, Sal866F-VIC | 7,5 |
| Sa0122, Sa0684, Sa1194 | Sa0122R, Sa0684R, Sa1194R | 11,25 |
| | Sa0122F, Sa0684F, Sa1194F | 7,5 |
| | Sa0122F-NED, Sa0684F-6FAM, Sa1194F-NED | 3,75 |
| Sa1729 | Sa1729R | 3,75 |
| | Sa1729F | 2,5 |
| | Sa1729F-6FAM | 1,25 |

**Tableau 4b : Constitution d'un mix d'amorces pour 100 typages (Panel 2) Un volume d'eau de 1172.5 µL est ajouté**

| Nom du VNTR | Nom de l'amorce | Volume d'amorce (à 20µM) à prélever |
|---|---|---|
| Sa2511 | Sa2511R | 22,5 |
| | Sa2511F | 15 |
| | Sa2511F-NED | 7,5 |
| Sa1132, Sa1291, Sa2039 | Sa1132R, Sa1291R, Sa2039R | 11,25 |
| | Sa1132F, Sa1291F, Sa2039F | 7,5 |
| | Sa1132F-6FAM, Sa1291F-NED, Sa2039F-6FAM | 3,75 |
| Sa0266, Sa0704 | Sa0266R, Sa0704R | 3,75 |
| | Sa0266F, Sa0704F | 2,5 |
| | Sa0266F-PET, Sa0704F-VIC | 1,25 |

Pour chaque réaction, le volume réactionnel est de 15µL dont 5,5µL de mix d'amorces, 7,5µL du mix QIAGEN Multiplex PCR 2X et 2 µL d'ADN bactérien (concentré à 2ng/µL). Le procédé d'extraction d'ADN ne fait pas l'objet de la présente invention mais les inventeurs recommandent l'utilisation du kit d'extraction DNeasy Blood & Tissue de Qiagen.

Chaque mélange réactionnel est ensuite soumis à une réaction de PCR dont le protocole original est indiqué sur le *Tableau 5* ci dessous. Puis, 7,5µL du produit PCR est purifié sur colonnes selon la technologie DyeEx de Qiagen. Enfin, la préparation d'un mélange constitué de 2µL de produit PCR purifié, de 7,75µL d'HiDi formamide (Applied Biosystems) et de 0,25µL de GS1200LIZ (Applied Biosystems) permet l'analyse des fragments par électrophorèse capillaire sur les plateformes Applied Biosystems Genetic Analyser 3100-Avant, 3130, 3130x1 et 3500. Le protocole d'électrophorèse capillaire est décrit sur le (*Tableau 6 ci-dessous.*

**Tableau 5 : Caractéristiques du protocole PCR utilisé**

| | Etape 1 1 fois | Etape 2 15 cycles | | | Etape 3 15 fois | | | Etape 4 1 fois |
|---|---|---|---|---|---|---|---|---|
| Temps | 15 min | 30 sec | 60 sec | 70 sec | 30 sec | 60 sec | 70 sec 70 (+5 sec/cycle) | 10 min |
| Température | 95 °C | 95°C | 75°C (-1°C /cycle) | 72°C | 95°C | 60°C | 72°C | 72 °C |

Le type de protocole PCR utilisé est ici la PCR par essais ou Touchdown PCR qui permet l'augmentation de la spécificité de l'amplification. Ce protocole PCR modifié évite les hybridations non spécifiques et réduit ainsi les artefacts d'amplification, particulièrement nuisibles lors de l'interprétation d'un profil électrophorétique obtenu selon le procédé décrit par la présente invention. Un protocole de PCR par essais consiste à démarrer le process d'amplification à une température d'hybridation très haute lors des premiers cycles afin d'assurer une forte stringence ; la séquence cible est ainsi amplifiée préférentiellement. La température d'hybridation est ensuite diminuée séquentiellement pour l'amener à une température proche du Tm des amorces afin d'assurer une meilleure efficacité de PCR.

**Tableau 6 : Caractéristiques du protocole d'électrophorèse capillaire utilisé (°C : degré Celsius, µA : micro ampère, kV: kilo volt, sec : seconde)**

| Caractéristiques | Valeurs utilisées |
|---|---|
| Run température | 60°C |
| Polymor fill volume | 5020 |
| Current stability | 5µA |
| Pre run voltage | 15kV |
| Pre run time | 180sec |
| Injection voltage | 1,6kV |
| Injection time | 10sec |
| Number of steps | 40 |
| Voltage step interval | 15sec |
| Data delay time | 80sec |
| Run voltage | 12kV |
| Run time | 6200sec |

### 5) Obtention du code MLVA

L'analyse du profil électrophorétique s'effectue par le logiciel GeneMapper^{®} qui interprète les données brutes et attribue une taille à chaque amplicon marqué par un fluorophore. Le présent demandeur a développé trois fichiers, issus de GeneMapper^{®}, nécessaires à l'attribution du code MLVA. Le fichier nommé « Méthode d'analyse » permet le traitement des données analysées selon des critères définis. Les fichiers nommés « MLVA management A» et « MLVA management B» rassemblent l'ensemble des données recueillies sur les différents allèles associés à chaque marqueur défini par une gamme de taille et de couleur. Ces fichiers sont illustrés en figures 10 et 11. L'export du «Report manager » sous forme d'un fichier .txt donne l'attribution d'une valeur numérique, reflet du nombre de répétitions, pour chaque marqueur VNTR. Cette valeur est associée à un indice de qualité indiquant le degré de confiance que l'utilisateur peut attribuer à ce résultat. Arbitrairement, un indice supérieur à 0,5 est considéré comme valide.

### EXEMPLES

### Exemple 1 :

Cet exemple illustre l'amplicon obtenu par amplification du VNTR Sa01223 avec les amorces SEQ ID NO: 1 et SEQ ID NO: 2 pour la souche *Staphylococcus aureus Mu50*
(SEQ ID NO: 33)

### Exemple 2 :

Cet exemple illustre l'amplicon obtenu par amplification du VNTR Sa0311 avec les amorces SEQ ID NO: 3 et SEQ ID NO: 4 pour la souche *Staphylococcus aureus Mu50*
(SEQ ID NO: 34)

### Exemple 3 :

Cet exemple illustre l'amplicon obtenu par amplification du VNTR Sa0387 avec les amorces SEQ ID NO: 5 et SEQ ID NO: 6 pour la souche *Staphylococcus aureus Mu50*
(SEQ ID NO: 35)

### Exemple 4 :

Cet exemple illustre l'amplicon obtenu par amplification du VNTR Sa0550 avec les amorces SEQ ID NO: 7 et SEQ ID NO: 8 pour la souche *Staphylococcus aureus Mu50*
(SEQ ID NO: 36)

### Exemple 5 :

Cet exemple illustre l'amplicon obtenu par amplification du VNTR Sa0684 avec les amorces SEQ ID NO: 9 et SEQ ID NO: 10 pour la souche *Staphylococcus aureus Mu50*
(SEQ ID NO: 37)

### Exemple 6 :

Cet exemple illustre l'amplicon obtenu par amplification du VNTR Sa0964 avec les amorces SEQ ID NO: 11 et SEQ ID NO: 12 pour la souche *Staphylococcus aureus Mu50*
(SEQ ID NO: 38)

### Exemple 7 :

Cet exemple illustre l'amplicon obtenu par amplification du VNTR Sa1097 avec les amorces SEQ ID NO: 13 et SEQ ID NO: 14 pour la souche *Staphylacoccus aureus Mu50*
(SEQ ID NO: 39)

### Exemple 8 :

Cet exemple illustre l'amplicon obtenu par amplification du VNTR Sa1194 avec les amorces SEQ ID NO: 15 et SEQ ID NO: 16 pour la souche *Staphylococcus aureus Mu50*
(SEQ ID NO: 40)

### Exemple 9 :

Cet exemple illustre l'amplicon obtenu par amplification du VNTR Sa1729 avec les amorces SEQ ID NO: 17 et SEQ ID NO: 18 pour la souche *Staphylococcus aureus Mu50*
(SEQ ID NO: 41)

### Exemple 10 :

Cet exemple illustre l'amplicon obtenu par amplification du VNTR Sa1866 avec les amorces SEQ ID NO: 19 et SEQ ID NO: 20 pour la souche *Staphylococcus aureus Mu50*
(SEQ ID NO: 42)

### Exemple 11 :

Cet exemple illustre l'amplicon obtenu par amplification du VNTR Sa0266 avec les amorces SEQ ID NO: 21 et SEQ ID NO: 22 pour la souche *Staphylococcus aureus Mu50*
(SEQ ID NO: 43)

### Exemple 12 :

Cet exemple illustre l'amplicon obtenu par amplification du VNTR Sa0704 avec les amorces SEQ ID NO: 23 et SEQ ID NO: 24 pour la souche *Staphylococcus aureus Mu50*
(SEQ ID NO: 44)

### Exemple 13 :

Cet exemple illustre l'amplicon obtenu par amplification du VNTR Sal132 avec les amorces SEQ ID NO: 25 et SEQ ID NO: 26 pour la souche *Staphylococcus aureus Mu50*
(SEQ ID NO: 45)

### Exemple 14 :

Cet exemple illustre l'amplicon obtenu par amplification du VNTR Sa1291 avec les amorces SEQ ID NO: 27 et SEQ ID NO: 28 pour la souche *Staphylococcus aureus Mu50*
(SEQ ID NO: 46)

### Exemple 15 :

Cet exemple illustre l'amplicon obtenu par amplification du VNTR Sa2039 avec les amorces SEQ ID NO: 29 et SEQ ID NO: 30 pour la souche *Staphylococcus aureus Mu50*
(SEQ ID NO: 47)

### Exemple 16 :

Cet exemple illustre l'amplicon obtenu par amplification du VNTR Sa2511 avec les amorces SEQ ID NO: 31 et SEQ ID NO: 32 pour la souche *Staphylococcus aureus Mu50*
(SEQ ID NO: 48)

### B) Test de génotypage

Le groupe d'étude sur les marqueurs épidémiologiques (l'ESGEM) a défini plusieurs critères permettant de définir la performance d'une méthode de typage. Une méthode fiable repose sur des marqueurs stables et identifiables sur les isolats typés. L'analyse des résultats de typage doit permettre une bonne discrimination entre les isolats tout en conservant une signification épidémiologique dans un contexte donné. Enfin, et de manière inhérente aux aspects expérimentaux, la technique considérée doit être reproductible afin d'envisager une future standardisation et automatisation. Cette méthode de typage doit être validée sur un panel de souches dont les caractéristiques doivent être connues. Ces souches doivent avoir été typées par d'autres méthodes afin de démontrer l'amélioration technologique.

### 1) Diversité des souches et indice de discrimination

Le traitement des résultats antérieurs de typage par MLVA (selon *Pourcel et al.* et *Schouls et al.)* et MLST (autre technique de l'art antérieur) a été réalisé par import des données numériques issues respectivement de Pourcel *et al.* et Schouls *et al.* dans le logiciel bionumerics v.6.0 (Applied Maths). Les ST ont été identifiés par Cookson *et al.,* chaque ST a été décomposé en un code numérique de 7 chiffres selon la description de chacun d'entre eux dans la base http://saureus.mlst.net.

Les résultats de typage développé dans la présente invention sont traités par le logiciel bionumerics v.6.0 (Applied Maths) à partir des données exportées de GeneMapper^{®} v.4.1 (Applied Biosystems).

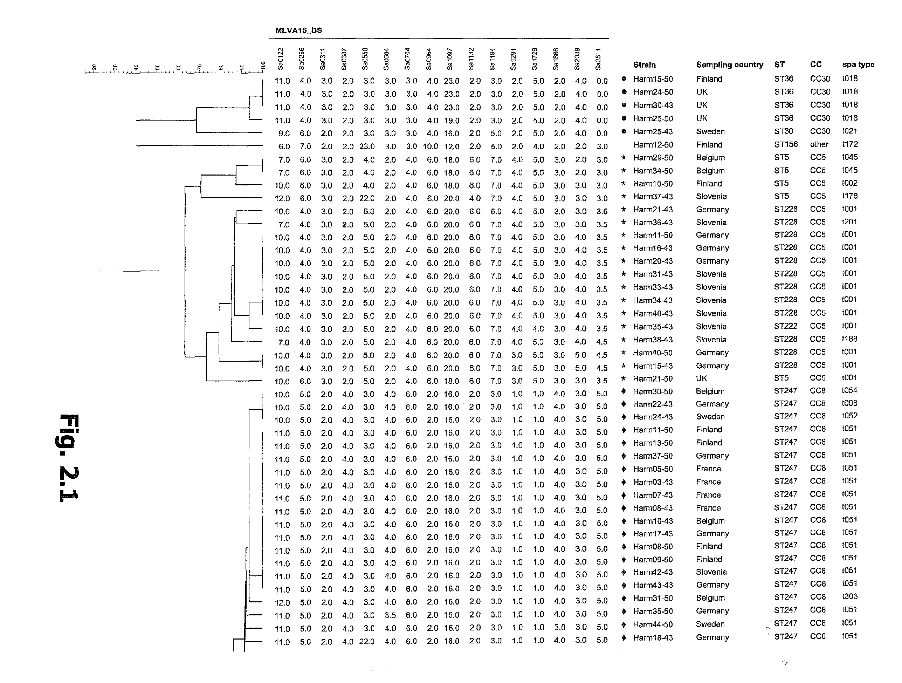

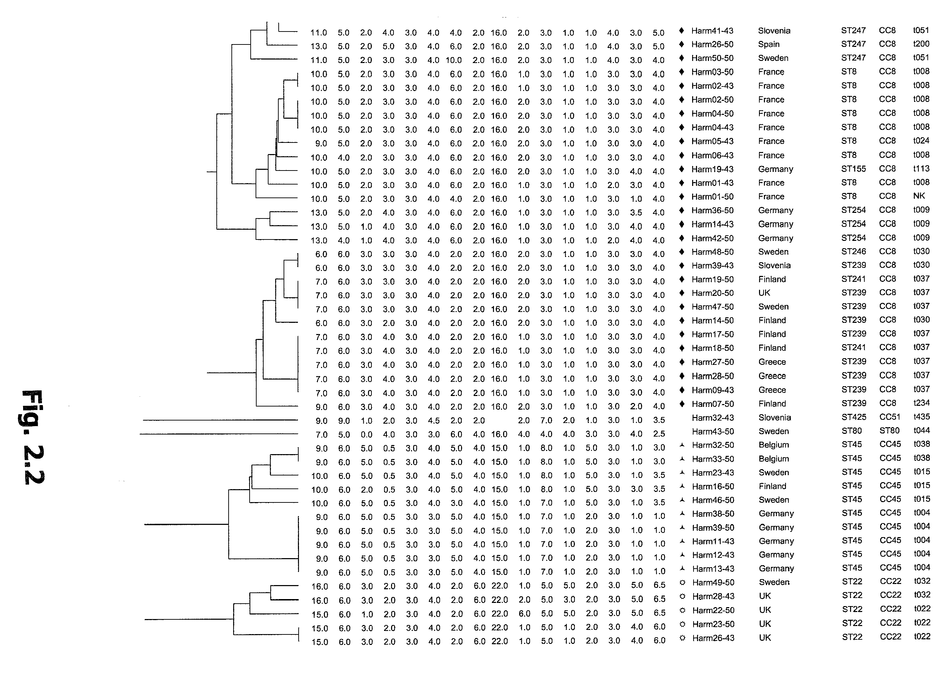

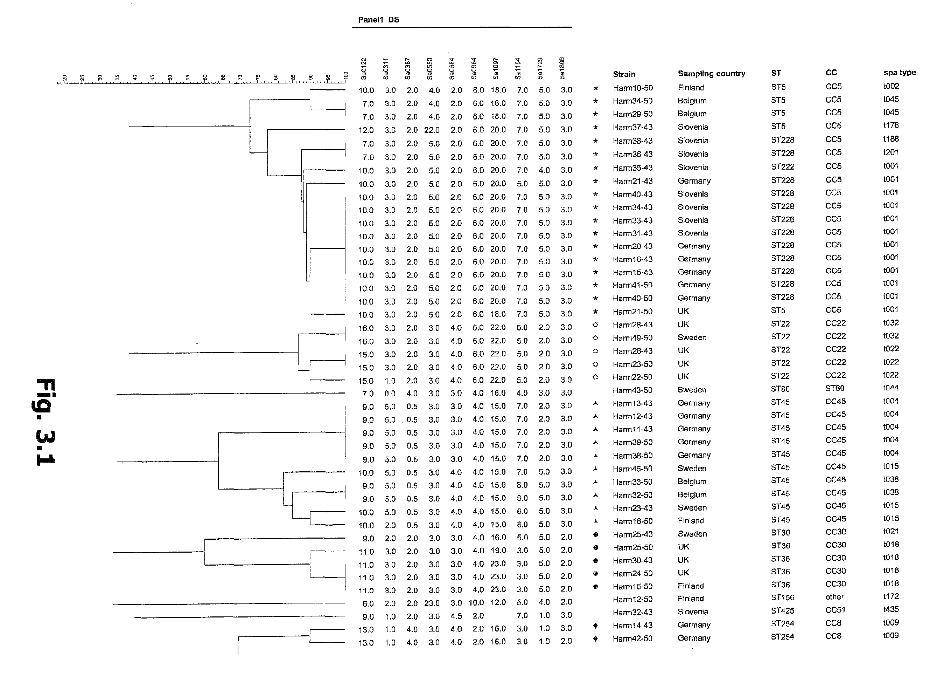

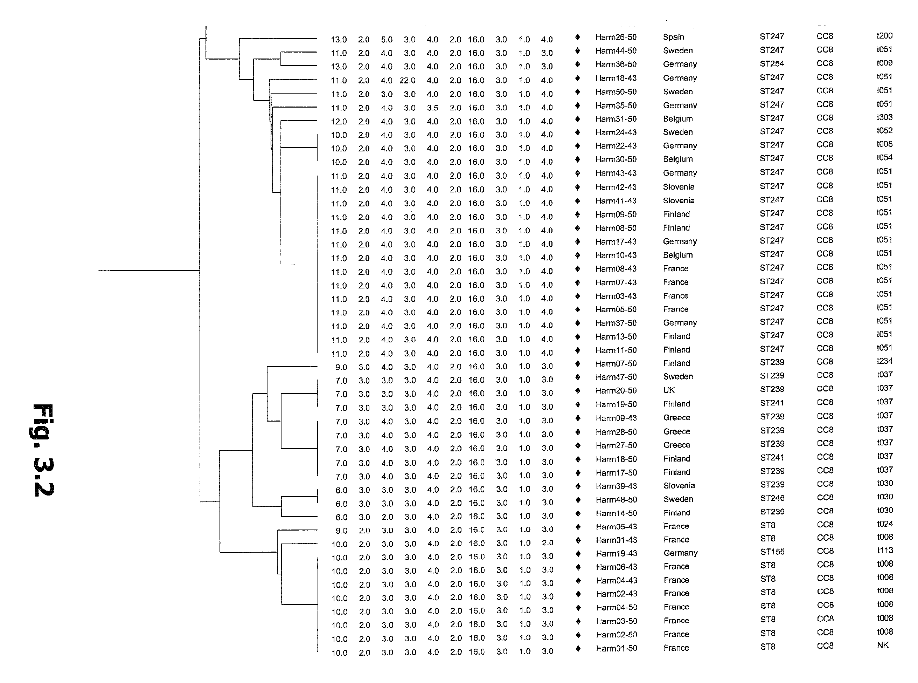

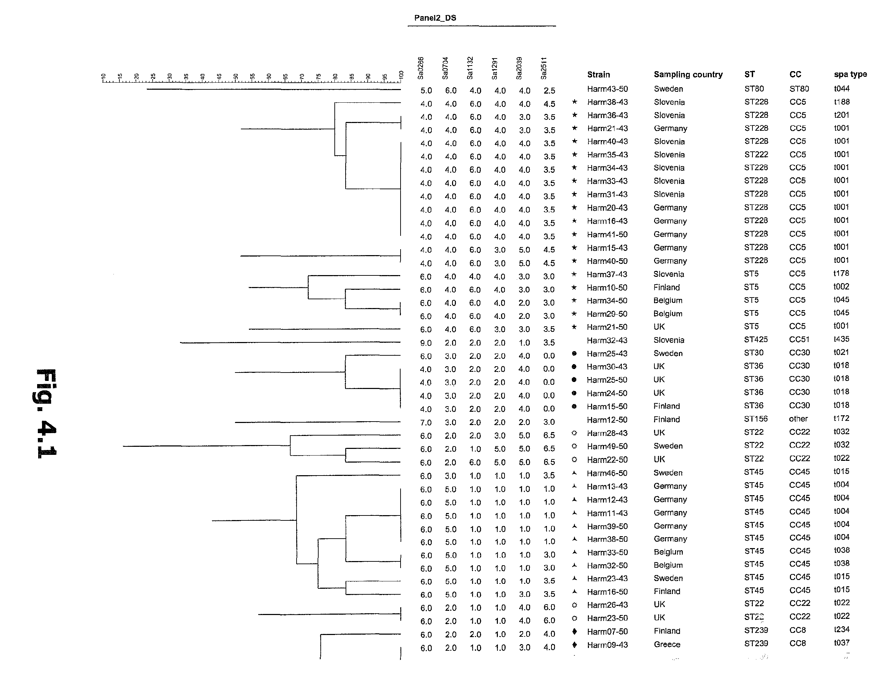

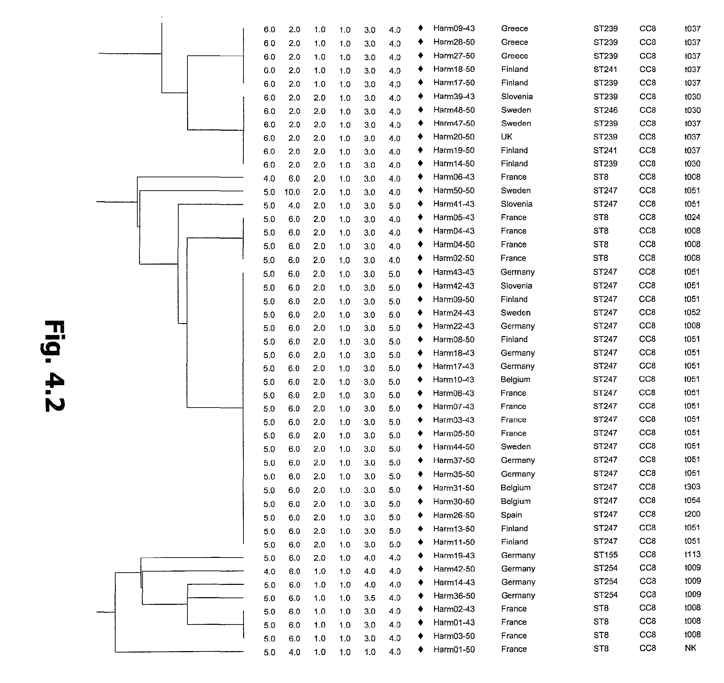

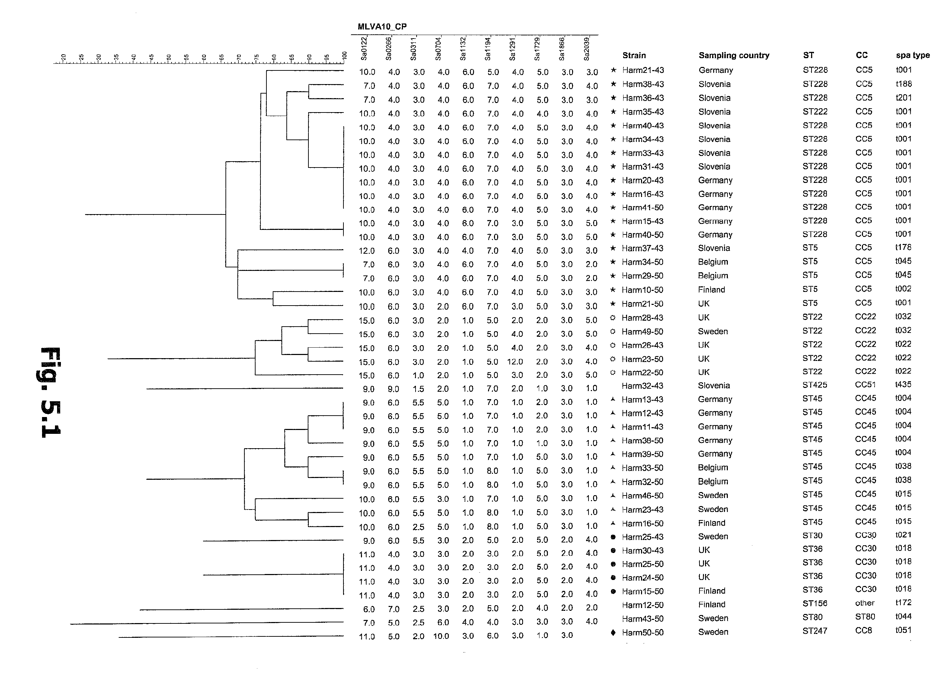

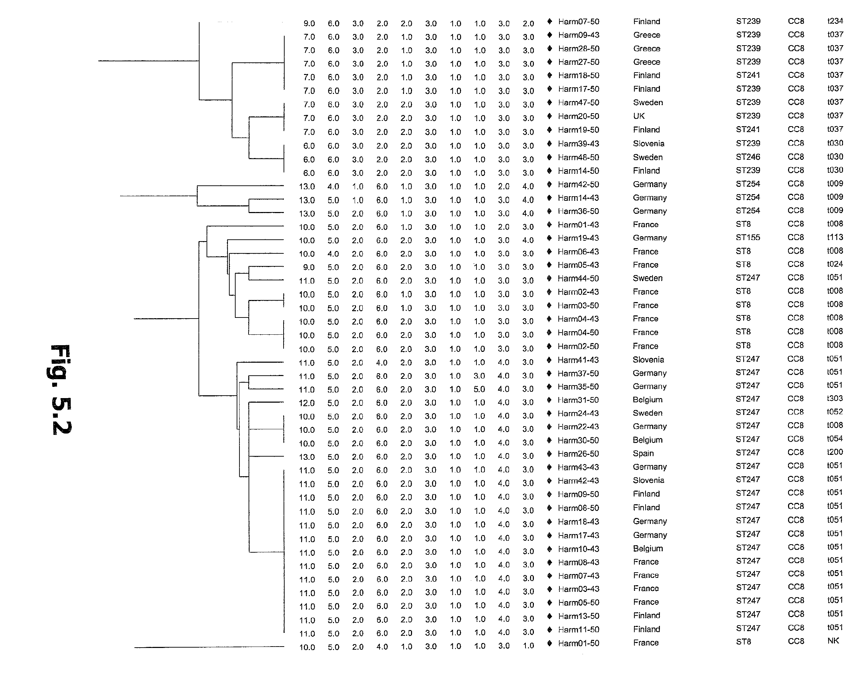

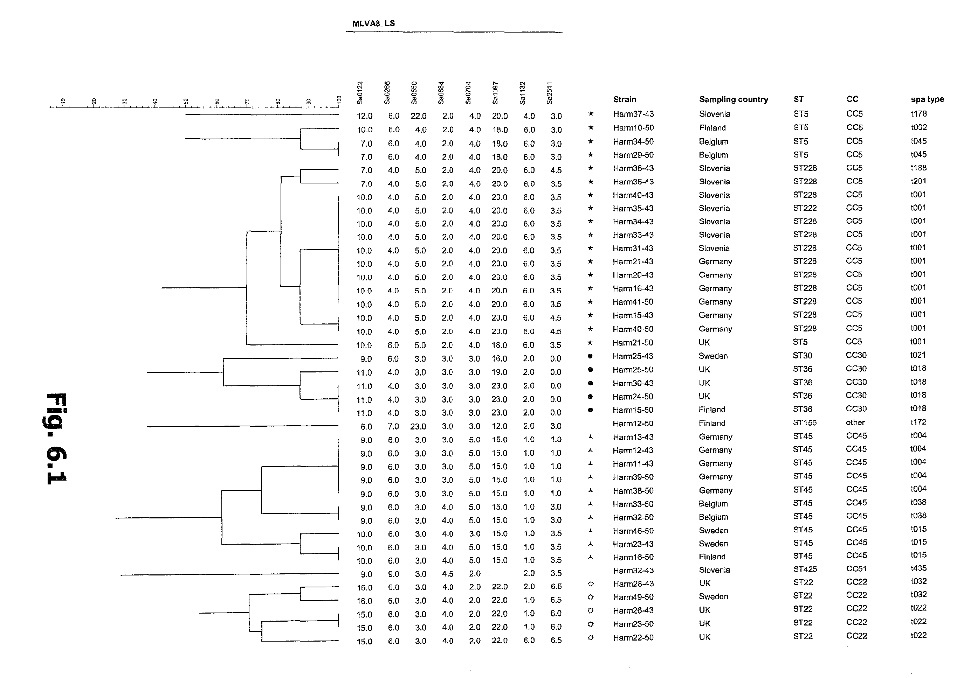

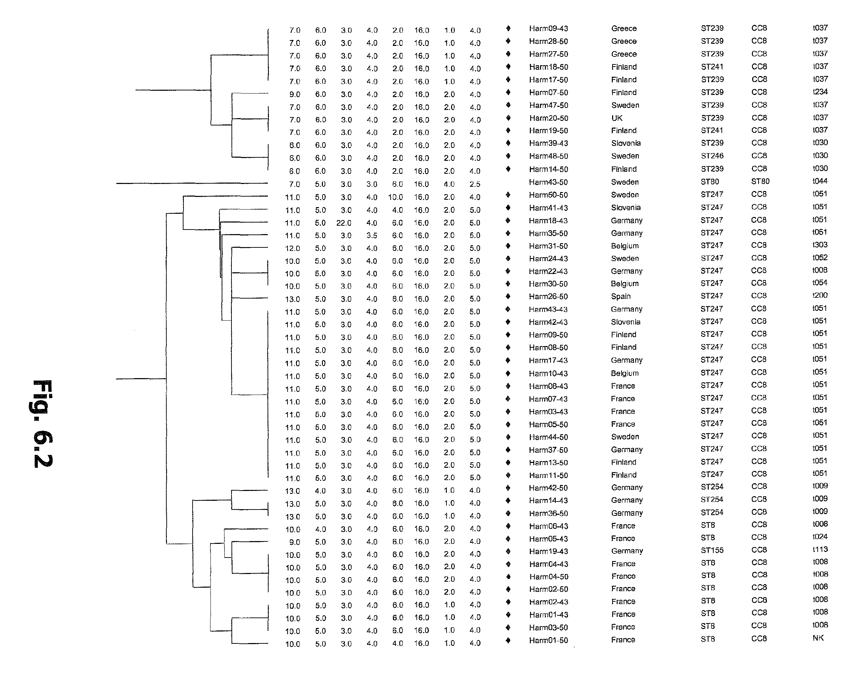

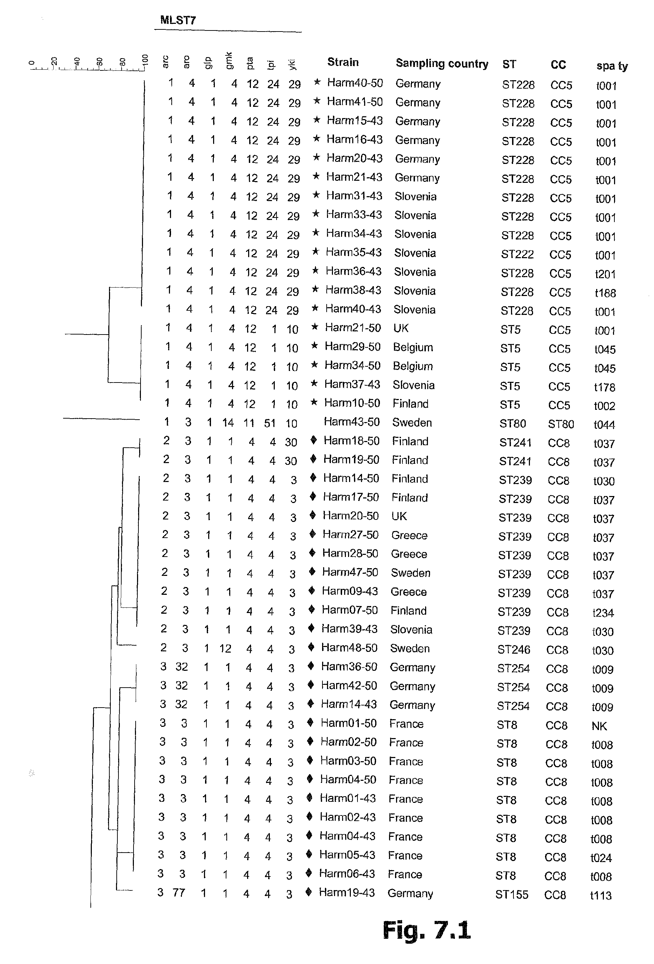

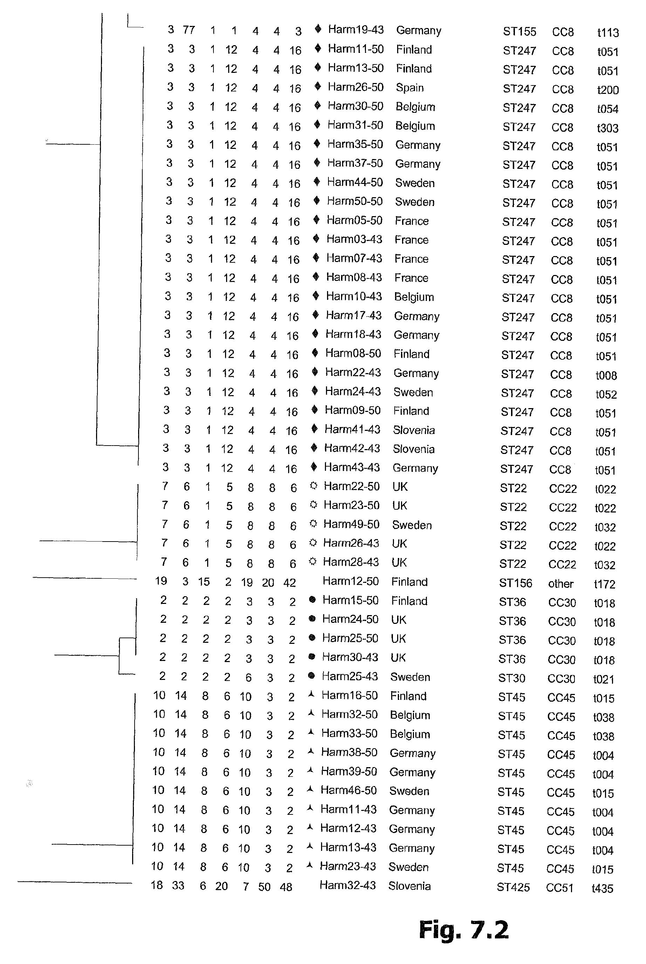

L'agrégation ou clustering a été obtenu par la méthode l'UPGMA selon le coefficient « categorical » (figures 2, 3, 4, 5, 6 et 7). Les motifs ont été imposés par un seuil « cut-off » de 60% sur l'agrégation ou « clustering » des données issues du typage décrit par la présente invention (appelée dans la suite de la description MLVA16_DS). Ces mêmes motifs ont été conservés pour les agrégations des données issues du présent procédé (Panel et Panel2), du protocole MLST (MLST), pour le MLVA décrit par Pourcel *et al.* (appelé ci-après MLVA10_CP) et pour le MLVA décrit par Schouls *et al.* (appelé ci-après MLVA8_LS). Ces symboles permettent de visualiser la corrélation entre les différents protocoles. Cette concordance entre les différents résultats de comparaison peut mathématiquement être évaluée par un indice dit de congruence calculé selon le coefficient de corrélation de Pearson.

Les protocoles MLVA (MLVA16_DS, MLVA10_CP et MLVA8_LS) sont très congruents (conservation des motifs) et montrent des indices de congruence très élevés. Ces indices sont indiqués sur le Tableau 7 ci après.

**Tableau 7 : Indice de congruence entre les différents protocoles**

| | MLVA16_DS | Panell_DS | MLST7 | MLVA8_LS | MLVA10_CP | Panel2_DS |
|---|---|---|---|---|---|---|
| MLVA16_DS | 100,00 | 97,01 | 92,61 | 92,80 | 92,51 | 89,10 |
| Panel1_DS | 97,01 | 100,00 | 91,76 | 86,29 | 85,70 | 75,95 |
| MLST7 | 92,61 | 91,76 | 100,00 | 79,04 | 81,17 | 77,85 |
| MLVA8_LS | 92,80 | 86,29 | 79,04 | 100,00 | 85,26 | 86,09 |
| MLVA10_CP | 92,51 | 85,70 | 81,17 | 85,26 | 100,00 | 86,55 |
| Panel2_DS | 89,10 | 75,95 | 77,85 | 86,09 | 86,55 | 100,00 |

Cette observation permet de conclure à la pertinence de chacun des VNTRs utilisés dans le procédé décrit par la présente invention. En effet, l'agencement des VNTRs entre eux et la combinaison des 16 VNTRs n'a pas eu d'effet sur la structure de l'arbre construit et sur la conservation des complexes clonaux. D'ailleurs, il est intéressant de remarquer que les protocoles MLVA, et notamment MLVA16_DS, sont congruents avec le MLST comme le prouve la conservation des motifs et les indices de congruence élevés.

Ces valeurs de congruence montrent que l'agencement des différents VNTRs au sein de chaque panel a été réalisé de manière pertinente. En effet, MLVA16_DS et Panel1_DS sont les protocoles les plus congruents avec le MLST. Le gain de congruence réalisé par l'ajout du panel 2 est minime (0,85%), l'analyse du panel 1 est donc nécessaire et suffisante pour classer correctement les groupes phylogénétiques.

Le calcul des indices de discrimination (D) a été déterminé, selon l'indice de Simpson, sur les 89 isolats appartenant à la collection HARMONY. Les indices de discrimination de MLVA16_DS, Panel1_DS, Panel2_DS, MLVA10_CP, MLVA8_LS et MLST7 sont respectivement de 0,965, 0,952, 0,925, 0,955, 0,955 et 0,878. Contrairement au MLST, les protocoles MLVA (excepté le panel 2) dont les pouvoirs discriminants sont sensiblement les mêmes, satisfont aux recommandations de l'ESGEM [ESCMID Study Group for Epidemiological Markers - ESCMID : European Society of Clinical Microbiology and Infectious Diseases] (D>0,95).

L'indice de discrimination élevé du panel 1 justifie qu'il puisse être utilisé seul lors d'investigations épidémiologiques. Cependant l'utilisation conjointe des deux panels assure une discrimination inégalée par les protocoles de typage précédemment développés.

Il est intéressant de remarquer que les 16 VNTRs utilisés présentent des indices de discrimination très différents (Tableau 8). Certains marqueurs comme Sa0550, Sal729 ou Sa1866 sont faiblement discriminants, permettant ainsi d'enraciner l'arbre et de consolider certains groupes phylogénétiques. Ces marqueurs sont d'ailleurs avantageusement compris dans le panel 1. D'autres VNTRs comme Sa0704, Sa1132 ou Sa2511 sont davantage discriminants et assurent la distinction génotypique d'isolats appartenant à de grandes familles. Ces marqueurs sont d'ailleurs avantageusement compris dans le panel 2.

**Tableau 8 : Diversité de la collection HARMONY et indices de discrimination des protocoles MLVA16 DS, Panel1, Panel2 ainsi que des VNTRs utilisés**

| Nom du VNTR | Nombre d'allèles | Indice de discrimination (D) | Intervalle de confiance de D à 95% |
|---|---|---|---|
| Sa0122 | 9 | 0,8062 | [0,7631 ; 0,8493] |
| Sa0266 | 5 | 0,6696 | [0,6333 ; 0,7058] |
| Sa0311 | 5 | 0,6397 | [0,5865 ; 0,6929] |
| Sa0387 | 5 | 0,7204 | [0,6802 ; 0,7605] |
| Sa0550 | 5 | 0,3767 | [0,2597 ; 0,4936] |
| Sa0684 | 5 | 0,5365 | [0,4389 ; 0,6341] |
| Sa0704 | 6 | 0,7546 | [0,7073 ; 0,8019] |
| Sa0964 | 4 | 0,6044 | [0,5334 ; 0,6755] |
| Sa1097 | 9 | 0,6476 | [0,5495 ; 0,7457] |
| Sa1132 | 4 | 0,6425 | [0,5852 ; 0,6998] |
| Sa1194 | 5 | 0,5822 | [0,4976 ; 0,6669] |
| Sa1291 | 5 | 0,5102 | [0,4003 ; 0,6202] |
| Sa1729 | 5 | 0,5983 | [0,5256 ; 0,6711] |
| Sa1866 | 3 | 0,4969 | [0,4036 ; 0,5903] |
| Sa2039 | 6 | 0,6453 | [0,5612 ; 0,7294] |
| Sa2511 | 10 | 0,8189 | [0,7775 ; 0,8604] |

| Protocole de typage | Nombre de génotypes | Indice de discrimination (D) | Intervalle de confiance de D à 95% |
|---|---|---|---|
| MLVA16_DS | 49 | 0,9650 | [0,9455 ; 0,9846] |
| Panel1 | 41 | 0,9515 | [0,9294 ; 0,9736] |
| Panel2 | 36 | 0,9252 | [0,8883 ; 0,9621] |

### 2) Typabilité

Le typage a été réalisé sur 89 isolats avec 16 VNTRs, 1424 données devraient donc avoir été produites. Or, 7 données n'ont pas pu être obtenues et sont indiquées par un 0 dans l'arbre (Figure 2). Le MLVA16_DS présente donc une typabilité de 99,6%, valeur très satisfaisante pour une méthode de typage (Tableau 9 ci-dessous).

**Tableau 9 : Typabilité des protocoles MLVA16 DS, Panel1, Panel2 ainsi que des VNTRs utilisés sur la collection HARMONY (89 isolats)**

| Nom du VNTR | Données manquantes | Typabilité (en%) |
|---|---|---|
| Sa0122 | 0 | 100 |
| Sa0266 | 0 | 100 |
| Sa0311 | 1 | 98,9 |
| Sa0387 | 0 | 100 |
| Sa0550 | 0 | 100 |
| Sa0684 | 0 | 100 |
| Sa0704 | 0 | 100 |
| Sa0964 | 0 | 100 |
| Sa1097 | 1 | 98,9 |
| Sa1132 | 0 | 100 |
| Sa1194 | 0 | 100 |
| Sa1291 | 0 | 100 |
| Sa1729 | 0 | 100 |
| Sa1866 | 0 | 100 |
| Sa2039 | 0 | 100 |
| Sa2511 | 5 | 94,4 |

| Protocole de typage | Données manquantes | Typabilité (en%) |
|---|---|---|
| MLVA16_DS | 7 | 99,6 |
| Panel1 | 2 | 99,8 |
| Panel2 | 5 | 99,1 |

### 3) Reproductibilité

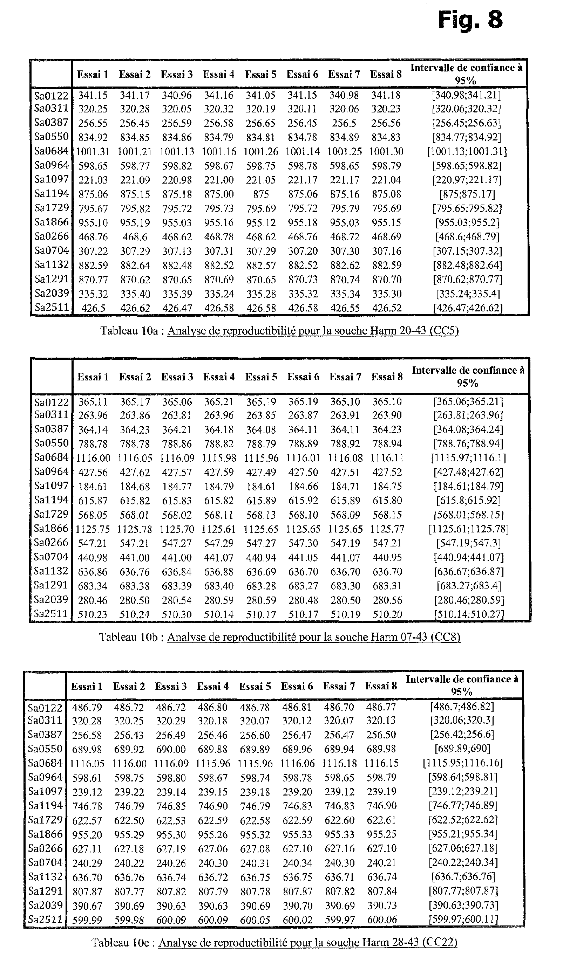

Les 89 isolats ont été typés deux fois et ont montré un même profil génotypique. Afin de valider cette reproductibilité, un plan expérimental précis a été mis en place. Ainsi, trois souches de la collection Harmony et appartenant à trois complexes clonaux différents ont été typées huit fois de manière indépendante. Les tailles observées de chaque locus pour les huit essais sont recensées sur les Tableaux 10a, 10b et 10c représentés en Figure 8. Un intervalle de confiance à 95% a été calculé selon un test de Student avec le coefficient de Student t(0.95 ; 8). Toutes les valeurs sont comprises dans leur intervalle de confiance respectif ; le typage décrit dans la présente invention est donc reproductible.

Bien que l'invention ait été décrite en liaison avec un mode de réalisation particulier, il est bien évident qu'elle n'y est nullement limitée et qu'elle comprend tous les équivalents techniques des moyens décrits ainsi que leurs combinaisons si celles-ci entrent dans le cadre de l'invention telle que définie dans les revendications.

## Revendications

1. Procédé de génotypage de l'espèce *Staphylococcus aureus* par MLVA comprenant les étapes consistant à :
(i) réaliser une première co-amplification de dix loci VNTRs par PCR multiplexe dans un premier milieu réactionnel comprenant un échantillon d'ADN issu d'un isolat de *Staphylococcus aureus* le premier milieu réactionnel comprenant les couples d'amorces respectivement anti-sens/sens suivants : SEQ ID NO :1 /SEQ ID NO :2 ; SEQ ID NO :3/ SEQ ID NO :4 ; SEQ ID NO :5/ SEQ ID NO :6 ; SEQ ID NO :7/ SEQ ID NO :8 ; SEQ ID NO :9/ SEQ ID NO : 10 ; SEQ ID NO : 11/ SEQ ID NO :12 ; SEQ ID NO :13/ SEQ ID NO :14 ; SEQ ID NO :15/ SEQ ID NO :16; SEQ ID NO : 17/ SEQ ID NO :18; SEQ ID NO : 19/ SEQ ID NO :20, afin de former plusieurs amplicons pour chacun des dix loci VNTRs,
puis une seconde co-amplification d'au moins six loci VNTRs supplémentaires par PCR multiplexe dans un second milieu réactionnel, chacun des premier et second milieux réactionnels comprenant un échantillon d'ADN issu du même isolat de *Staphylococcus aureus,* le second milieu réactionnel comprenant les couples d'amorces respectivement anti-sens/sens suivants : SEQ ID NO : 21/ SEQ ID NO : 22; SEQ ID NO : 23/ SEQ ID NO : 24; SEQ ID NO : 25/ SEQ ID NO : 26; SEQ ID NQ : 27/ SEQ ID NO : 28; SEQ ID NO : 29/ SEQ ID NO : 30 et SEQ ID NO : 31/ SEQ ID NO : 32,
(ii) analyser lesdits amplicons par électrophorèse capillaire afin d'obtenir un profil électrophorétique dudit échantillon, et
(iii) attribuer un code numérique MLVA audit profil électrophorétique.

2. Procédé de génotypage selon la revendication 1, dans lequel le code numérique MLVA de l'étape (iii) est obtenu automatiquement par informatique, à partir d'un logiciel d'analyse de fragments.

3. Procédé de génotypage selon la revendication 1 ou 2, dans lequel les amorces sens sont marquées par avec un fluorophore défini par un spectre d'excitation unique permettant d'associer un VNTR donné à une gamme de couleur ou de motifs lors de la visualisation des profils électrophorétiques.

4. Procédé de génotypage selon l'une quelconque des revendications précédentes **caractérisé en ce que** le milieu réactionnel comprend en outre des amorces sens non-marquées, les séquences des amorces sens marquées et non marquées étant identiques, selon un ratio [amorces sens non marquées] / [amorces anti-sens] = 2/3 et [amorces sens marquées] / [amorces anti-sens] = 1/3.

5. Kit de génotypage de *Staphylococcus aureus* pour la mise en oeuvre du procédé de génotypage selon l'une des revendications 1 à 4, comprenant :
- un premier milieu réactionnel comportant dix amorces sens marquées et dix amorces anti-sens, formant dix couples d'amorces sens/anti-sens pour chacun desquels correspond un locus VNTR spécifique et différent pour chacun des dix couples d'amorces, chacune des dix amorces sens s'hybridant en amont de son locus respectif et chacune des dix amorces anti-sens s'hybridant en aval de son locus respectif, les couples d'amorces respectivement anti-sens/sens étant les suivants : SEQ ID NO :1 /SEQ ID NO :2 ; SEQ ID NO :3/ SEQ ID NO :4 ; SEQ ID NO :5/ SEQ ID NO :6 ; SEQ ID NO :7/ SEQ ID NO :8 ; SEQ ID NO :9/ SEQ ID NO :10 ; SEQ ID NO :11/ SEQ ID NO :12 ; SEQ ID NO :13/ SEQ ID NO :14 ; SEQ ID NO :15/ SEQ ID NO :16; SEQ ID NO : 17/ SEQ ID NO :18; SEQ ID NO : 19/ SEQ ID NO :20,
- un second milieu réactionnel comprenant les couples d'amorces respectivement anti-sens/sens suivants : SEQ ID NO : 21/ SEQ ID NO : 22; SEQ ID NO : 23/ SEQ ID NO : 24; SEQ ID NO : 25/ SEQ ID NO : 26; SEQ ID NO : 27/ SEQ ID NO : 28; SEQ ID NO : 29/ SEQ ID NO : 30 et SEQ ID NO : 31/ SEQ ID NO : 32.
- un mix d'amplification,
- un témoin positif et un témoin négatif.

## Patentansprüche

1. Verfahren zur Genotypisierung der Art *Staphylococcus aureus* durch MLVA, das die Schritte umfasst, die darin bestehen:
(i) Durchführen einer ersten Co-Amplifikation von mindestens zehn VNTRs-Loci durch multiplexe PCR in einem ersten Reaktionsmedium, das eine DNA-Probe aus einem Isolat von *Staphylococcus aureus* umfasst, wobei das erste Redaktionsmedium die jeweiligen folgenden Anti-Sense/Sense-Primerpaare umfasst: SEQ ID NO: 1/SEQ ID NO: 2; SEQ ID NO: 3/SEQ ID NO: 4; SEQ ID NO: 5/SEQ ID NO: 6; SEQ ID NO: 7/SEQ ID NO: 8; SEQ ID NO: 9/SEQ ID NO: 10; SEQ ID NO: 11/SEQ ID NO: 12; SEQ ID NO: 13/SEQ ID NO: 14; SEQ ID NO: 15/SEQ ID NO: 16; SEQ ID NO: 17/SEQ ID NO: 18; SEQ ID NO: 19/SEQ ID NO: 20, um für jeden der zehn VNTRs-Loci mehrere Amplikone zu bilden,
gefolgt von einer zweiten Co-Amplifikation von mindestens zehn zusätzlichen VNTRs-Loci durch multiplexe PCR in einem zweiten Reaktionsmedium, wobei jedes des ersten und zweiten Reaktionsmediums eine DNA-Probe aus demselben Isolat von *Staphylococcus aureus* umfasst, wobei das zweite Redaktionsmedium die jeweiligen folgenden Anti-Sense/Sense-Primerpaare umfasst: SEQ ID NO: 21/SEQ ID NO: 22; SEQ ID NO: 23/SEQ ID NO: 24; SEQ ID NO: 25/SEQ ID NO: 26; SEQ ID NO: 27/SEQ ID NO: 28; SEQ ID NO: 29/SEQ ID NO: 30 und SEQ ID NO: 31/SEQ ID NO: 32,
(ii) Analysieren der Amplikone durch Kapillarelektrophorese, um ein Elektrophoreseprofil der Probe zu erhalten, und
(iii) Zuweisen eines numerischen MLVA-Codes an das Elektrophoreseprofil.

2. Verfahren zur Genotypisierung nach Anspruch 1, wobei der numerische MLVA-Code von Schritt (iii) automatisch durch Informatik mit einer Fragmentanalysesoftware erhalten wird.

3. Verfahren zur Genotypisierung nach Anspruch 1 oder 2, wobei die Sense-Primer von einem Fluorophor markiert sind, das von einem einzigen Erregungsspektrum definiert ist, das erlaubt, einen bestimmten VNTR einer Farb- oder Motivenskala bei der Visualisierung der Elektrophoreseprofile zuzuordnen.

4. Verfahren zur Genotypisierung nach eine der vorangehenden Ansprüche, **dadurch gekennzeichnet, dass** das Reaktionsmedium ferner nicht markierte Sense-Primer umfasst, wobei die Sequenzen der markierten und nicht markierten Sense-Primer gemäß einem Verhältnis [nicht markierte Anti-Sense-Primer]/[Sense-Primer] = 2/3 und [markierte Sense-Primer]/[Anti-Sense-Primer] = 1/3 identisch sind.

5. Kit zur Genotypisierung von *Staphylococcus aureus* für die Umsetzung des Verfahrens zur Genotypisierung nach einem der Ansprüche 1 bis 4, umfassend:
- ein erstes Reaktionsmedium, das zehn markierte Sense-Primer und zehn Anti-Sense-Primer aufweist, die zehn Sense-/Anti-Sense-Primerpaare bilden, wobei jedem von ihnen ein spezieller VNTR-Locus entspricht, der für jedes der zehn Primerpaare unterschiedlich ist, wobei jeder der zehn Sense-Primer vorgelagert von seinem jeweiligen Locus hybridisiert und jeder der zehn Anti-Sense-Primer nachgelagert von seinem jeweiligen Locus hybridisiert, wobei die jeweiligen Anti-Sense-/Sense-Primerpaare folgende sind: SEQ ID NO: 1/SEQ ID NO: 2; SEQ ID NO: 3/SEQ ID NO: 4; SEQ ID NO: 5/SEQ ID NO: 6; SEQ ID NO: 7/SEQ ID NO: 8; SEQ ID NO: 9/SEQ ID NO: 10; SEQ ID NO: 11/SEQ ID NO: 12; SEQ ID NO: 13/SEQ ID NO: 14; SEQ ID NO: 15/SEQ ID NO: 16; SEQ ID NO: 17/SEQ ID NO: 18; SEQ ID NO: 19/SEQ ID NO: 20,
- ein zweites Redaktionsmedium, das die jeweiligen folgenden Anti-Sense/Sense-Primerpaare umfasst: SEQ ID NO: 21/SEQ ID NO: 22; SEQ ID NO: 23/SEQ ID NO: 24; SEQ ID NO: 25/SEQ ID NO: 26; SEQ ID NO: 27/SEQ ID NO: 28; SEQ ID NO: 29/SEQ ID NO: 30 und SEQ ID NO: 31/SEQ ID NO: 32,
- einen Amplifikationsmix,
- eine positive Kontrolle und eine negative Kontrolle.

## Claims

1. A method for genotyping the species *Staphylococcus aureus* using MLVA, comprising the steps of:
(i) performing a first co-amplification of ten VNTR loci by multiplex PCR in a first reaction medium comprising a DNA sample derived from an isolate of *Staphylococcus aureus,* the first reaction medium comprising the following respective antisense/sense primer pairs: SEQ ID NO:1/SEQ ID NO:2; SEQ ID NO:3/SEQ ID NO:4; SEQ ID NO:5/SEQ ID NO:6; SEQ ID NO:7/SEQ ID NO:8; SEQ ID NO:9/SEQ ID NO:10; SEQ ID NO:11/SEQ ID NO:12; SEQ ID NO:13/SEQ ID NO:14; SEQ ID NO:15/SEQ ID NO:16; SEQ ID NO: 17/SEQ ID NO: 18; SEQ ID NO: 19/SEQ ID NO:20 to form several amplicons for each of the ten VNTR loci.
followed by a second co-amplification of at least six additional VNTR loci by multiplex PCR in a second reaction medium, each of the first and second reaction media comprising a DNA sample derived from the same isolate of *Staphylococcus aureus,* the second reaction medium comprising the following respective antisense/sense primer pairs: SEQ ID NO: 21/SEQ ID NO:22; SEQ ID NO:23/SEQ ID NO:24; SEQ ID NO:25/SEQ ID NO: 26; SEQ ID NO:27/SEQ ID NO:28; SEQ ID NO: 29/SEQ ID NO:30 and SEQ ID NO: 31/SEQ ID NO: 32;
(ii) analysing the said amplicons by capillary electrophoresis to obtain an electrophoretic profile of the said sample; and
(iii) allocating a numerical MLVA code to the said electrophoretic profile.

2. The genotyping method according to claim 1 wherein the numerical MLVA code at step (iii) is automatically obtained by data processing using fragment analysis software.

3. The genotyping method according to claim 1 or 2 wherein the sense primers are labelled with a fluorophore defined by a unique excitation spectrum allowing the association of a given VNTR with a colour or pattern range when visualizing the electrophoretic profiles.

4. The genotyping method according to any of the preceding claims, **characterized in that** the reaction medium further comprises non-labelled sense primers, the sequences of the labelled and non-labelled sense primers being identical with a ratio of [non-labelled sense primers]/[anti-sense primers] = 2:3 and [labelled sense primers] / [anti-sense primers] = 1:3.

5. A kit for the genotyping of *Staphylococcus aureus* to apply the genotyping method according to one of claims 1 to 4, containing:
- a first reaction medium comprising ten labelled sense primers and ten antisense primers forming ten pairs of sense/antisense primers for each of which there is a specific corresponding VNTR locus that is different for each of the ten primer pairs, each of the ten sense primers hybridizing upstream of its respective locus and each of the ten antisense primers hybridizing downstream of its respective locus, the pairs of respective antisense/sense primers being the following: SEQ ID NO:1/SEQ ID NO:2; SEQ ID NO:3/SEQ ID NO:4; SEQ ID NO:5/SEQ ID NO:6; SEQ ID NO:7/SEQ ID NO:8; SEQ ID NO:9/SEQ ID NO:10; SEQ ID NO:11/SEQ ID NO:12; SEQ ID NO:13/SEQ ID NO:14; SEQ ID NO:15/SEQ ID NO:16; SEQ ID NO: 17/SEQ ID NO: 18; SEQ ID NO: 19/SEQ ID NO:20;
- a second reaction medium comprising the following respective antisense/sense primer pairs: SEQ ID NO: 21/SEQ ID NO: 22; SEQ ID NO: 23/SEQ ID NO: 24; SEQ ID NO: 25/SEQ ID NO: 26; SEQ ID NO: 27/SEQ ID NO: 28; SEQ ID NO: 29/SEQ ID NO: 30 and SEQ ID NO: 31/SEQ ID NO: 32.
∘ an amplification mix
∘ a positive control and a negative control.
